(19) Europäisches Patentamt
European Patent Office
Office européen des brevets

(11) EP 4 674 431 A1

(12) **EUROPEAN PATENT APPLICATION**
published in accordance with Art. 153(4) EPC

(43) Date of publication:
**07.01.2026 Bulletin 2026/02**

(21) Application number: **24764057.6**

(22) Date of filing: **04.03.2024**

(51) International Patent Classification (IPC):
***A61K 39/395*** *(2006.01)* ***A61K 9/51*** *(2006.01)*
***A61K 9/127*** *(2025.01)* ***A61K 47/14*** *(2017.01)*
***A61K 47/24*** *(2006.01)* ***A61K 47/28*** *(2006.01)*
***A61K 47/36*** *(2006.01)* ***A61K 47/42*** *(2017.01)*
***A61P 35/00*** *(2006.01)* ***A61P 43/00*** *(2006.01)*

(52) Cooperative Patent Classification (CPC):
**A61K 9/127; A61K 9/51; A61K 39/395; A61K 47/14; A61K 47/24; A61K 47/28; A61K 47/36; A61K 47/42; A61P 35/00; A61P 43/00**

(86) International application number:
**PCT/JP2024/008154**

(87) International publication number:
**WO 2024/181580 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.03.2023 JP 2023031510**

(71) Applicant: **Japan Science and Technology Agency**
**Kawaguchi-shi**
**Saitama 332-0012 (JP)**

(72) Inventors:
- **KAWAGUCHI Yoshimasa**
  **Uji-city, Kyoto 6110011 (JP)**
- **FUTAKI Shiroh**
  **Uji-city, Kyoto 6110011 (JP)**
- **HIRAI Yusuke**
  **Uji-city, Kyoto 6110011 (JP)**
- **HIROSE Hisaaki**
  **Uji-city, Kyoto 6110011 (JP)**
- **KASAHARA Chisato**
  **Uji-city, Kyoto 6110011 (JP)**

(74) Representative: **Grünecker Patent- und Rechtsanwälte**
**PartG mbB**
**Leopoldstraße 4**
**80802 München (DE)**

# (54) LIPID NANOPARTICLES, PHARMACEUTICAL COMPOSITION, AND PRODUCTION METHOD FOR LIPID NANOPARTICLES

(57) The present disclosure is a lipid nanoparticle including a composition including a protein selected from an antibody and an antibody fragment, a polymer, and a lipid, in which the polymer and the protein have opposite charges at a predetermined pH. The lipid nanoparticle preferably has a protein encapsulated therein. In addition, when the particle diameter of the lipid nanoparticles is measured, the peak top of the particle diameter distribution is preferably at 80 nm to 120 nm.

[FIG. 1]

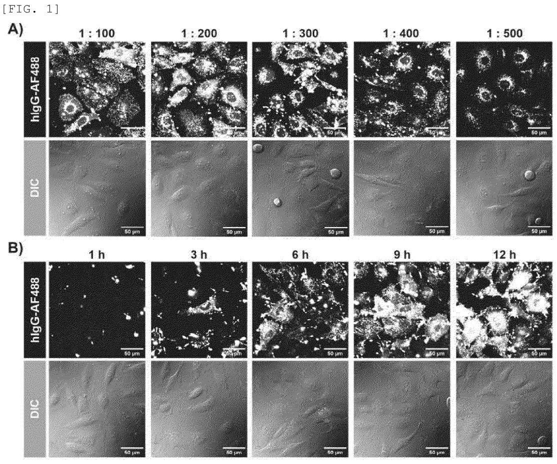


EP 4 674 431 A1

## Description

### Technical Field

The present disclosure relates to a lipid nanoparticle. In particular, the present disclosure relates to a lipid nanoparticle in which proteins such as antibodies can be encapsulated in the lipid nanoparticle and can be delivered into a cell including a cytosol (cytoplasmic matrix), a pharmaceutical composition, and a method for producing the lipid nanoparticle.

### Background Art

Since antibody therapeutics have antigen-specific binding ability, they are put into practical use not only as research reagents but also as molecular targettherapeutics, and are biopharmaceuticals that play important roles in modern medical care. However, antibodies are highly hydrophilic macromolecules (approximately 150 kDa) and their targets are limited to extracellular elements due to limitations of biological membrane permeability. That is, when a groundbreaking method for introducing antibodies into cells can be developed, the application range of antibodies can be expanded from extracellular antigens to intracellular antigens, and new disease applications of antibody therapeutics can be expected.

The lipid nanoparticle (LNP) technology represented by COVID-19 vaccines (Comirnaty (registered trademark) and Spikevax (registered trademark)) is capable of delivering nucleic acid molecules such as mRNA into target cells, and has expanded the application range of biopharmaceuticals. LNP is a lipid nanoparticle formulation that efficiently encapsulates negatively charged molecules such as nucleic acids and enhances their delivery to the cytosol, and is also expected to be applied to antibody therapeutics.

Non Patent Literature 1 describes that DOSP:MM27 (imidazole-based helper lipid MM27 + aminoglycoside lipid dioleyl succinyl paromomycin (DOSP)), which is a liposome-based formulation, was able to deliver an anti-cytokeratin 8 (K8) antibody into cells.

Non Patent Literature 2 describes that antibodies against α-tubulin and β-actin were delivered into cells using Lipospermine DOGS (dioctadecylglycylspermine) dissolved in ethanol.

Patent Literature 1 describes a method for concentrating a protein by forming a complex between a protein having a surface charge and a polyamino acid in a buffer solution.

Patent Literature 2 describes lipids that form lipid nanoparticles used in drug delivery systems. The use of this lipid is considered to improve the ability to escape from the endosome after endocytosis.

### Citation List

#### Patent Literature

Patent Literature 1: WO 2015/064591 A
Patent Literature 2: WO 2019/188867 A

Non Patent Literature 1: Chatin B, Mevel M, Devalliere J, et al. Liposome-based formulation for intracellular delivery of functional proteins. Mol Ther Nucleic Acids. 2015;4:e244
Non Patent Literature 2: Mol Ther. 2004 Jun;9(6):964-9. doi:10.1016/j.ymthe.2004.03.007.Intracytoplasmic delivery of anionic proteins

### Summary of Invention

#### Technical Problem

An object of the present disclosure is to deliver useful proteins into cells, particularly to deliver antibodies against intracellular functional proteins into cells, such as into cancer cells.

#### Solution to Problem

The present inventors have found that, in encapsulating proteins, which are polymers having multivalent charges, into lipids, the coexistence of an acidic polymer such as an acidic polyamino acid results in the generation of

lipid nanoparticles with smaller particle diameters, and thus completed the present disclosure.

(1) A lipid nanoparticle comprising a composition comprising a protein selected from an antibody and an antibody fragment, a polymer, and a lipid, wherein the protein and the polymer have opposite charges at a predetermined pH.
(2) The lipid nanoparticle according to (1), wherein the protein is encapsulated in the lipid nanoparticle.
(3) The lipid nanoparticle according to (1) or (2), wherein a peak top of a particle diameter distribution is at 80 to 120 nm when a particle diameter of the lipid nanoparticle is measured.
(4) The lipid nanoparticle according to any one of (1) to (3), wherein the antibody or antibody fragment specifically binds to a polypeptide, a peptide, a gene, or a lipid or a sugar chain bound to the polypeptide, the peptide, the gene, or the lipid.
(5) The lipid nanoparticle according to any one of (1) to (4), wherein the polymer is an anionic or cationic polymer.
(6) The lipid nanoparticle according to (5),
wherein the cationic polymer is at least one selected from the group consisting of polylysine, polyarginine, polyhistidine, and water-soluble salts thereof.
(7) The lipid nanoparticle according to (5),
wherein the anionic polymer is at least one polyamino acid selected from the group consisting of polyglutamic acid and polyaspartic acid, or at least one glycosaminoglycan selected from the group consisting of hyaluronic acid and pullulan.
(8) The lipid nanoparticle according to (5),
wherein the cationic or anionic polyamino acid has a molecular weight of 0.5 kDa to 1,000 kDa.
(9) The lipid nanoparticle according to any one of (1) to (8), wherein the lipid is a cationic lipid.
(10) The lipid nanoparticle according to (9),
wherein the cationic lipid is an intracellular environmentally responsive lipid having one or more properties selected from an ionizable property, a reducing environment responsive property, and a self-degradable property.
(11) The lipid nanoparticle according to any one of (1) to (10), further comprising one or more lipids selected from the group consisting of a neutral phospholipid, a PEG lipid, and cholesterol.
(12) A pharmaceutical composition comprising a composition comprising a protein selected from an antibody and an antibody fragment, a polymer, and a lipid, wherein the protein and the polymer have opposite charges at a predetermined pH.
(12-1) A method for delivering an antibody that is a pharmaceutically active ingredient, comprising administering the pharmaceutical composition according to the above (12).
(12-2) Use of the pharmaceutical composition according to the above (12).
(13) A method for producing a lipid
nanoparticlecomprising:

a droplet forming step of mixing a protein selected from the antibody and the antibody fragment with the polymer to form a droplet; and
an encapsulation step of mixing the droplet and the lipid to encapsulate the droplet with the lipid.

(13-1) The method for producing a lipid nanoparticle according to (13), wherein at least one step selected from the droplet forming step and the encapsulation step is a step of using a flow path of a microfluidic device.
(14) A lipid nanoparticle comprising a composition comprising a protein, a polymer, and a lipid, wherein the polymer has an opposite charge to that of the protein at a certain pH.

Advantageous Effects of Invention

According to the present disclosure, a protein selected from an antibody and an antibody fragment can be delivered into a cell including the cytoplasm and the nucleus.

Brief Description of Drawings

Fig. 1A) is a view illustrating that 1:200 IgG_polyE-LNP delivered IgG to a cytosol most efficiently. Fig. 1B) is a view illustrating that, in the case of the 1:200 IgG_polyE-LNP, changes over time were observed, and IgG was delivered to the cytosol 6 hours after addition.
Fig. 2A) is a view illustrating intracellular localization of IgG when hIgG-AF488_polyE-LNP was added to serum-containing medium at a concentration of 1 μM in HeLa cells. Fig. 2B) is a view illustrating that an Alexa Fluor 488 signal

present in the cytosol is due to the delivery of IgG molecules, as an IgG-AF488 signal in the cytosol was colocalized with the signal of an anti-hIgG secondary antibody.

Fig. 3A) is a view showing that, when observing the intracellular localization of hIgG-AF488, the fluorescence signal of IgG_polyE-LNP observed in the cytosol is clearly higher than that of IgG-LNP. Fig. 3B) is a view illustrating that, when the amount of antibody transferred into cells by each LNP is quantified by flow cytometry, the intracellular uptake amount of IgG_polyE-LNP was approximately two times that of IgG-LNP.

Fig. 4 is a view illustrating that cells to which IgG_polyE-LNP was added exhibited five times or more the luminescence compared to IgG-LNP.

Fig. 5A) is a view illustrating that, when anti-GFP-IgG-AF594 was delivered to the cytosol by IgG_polyE-LNP, GFP fluorescence and AF594 fluorescence in a fusion protein of HRas(G12V) and GFP expressed in the cell was colocalized, indicating that the fluorescently labeled antibody was delivered to the cytosol while maintaining its antigen recognition ability. Fig. 5B) is a view illustrating that, when anti-GFP-IgG was delivered to the cytosol by IgG_polyE-LNP, and the cells were fixed and permeabilized, and stained with Alexa594-labeled anti-mouse IgG secondary antibody, the GFP fluorescence and Alexa594 fluorescence in a fusion protein of HRas(G12V) and GFP expressed in the cell was colocalized, indicating that the non-fluorescently labeled antibody was delivered to the cytosol while maintaining its antigen recognition ability.

Fig. 6A) is a view illustrating that, when anti-NPC-IgG was delivered to the cytosol by IgG_polyE-LNP and immunostained with Alexa594-labeled anti-mouse IgG secondary antibody, AF594 fluorescence was accumulated around the nuclei stained with Hoechst, indicating that a nuclear pore complex was recognized. Fig. 6B) is a view confirming that, by line plot analysis, the AF594 fluorescence signal was accumulated at the periphery of the Hoechst fluorescence signal that labels the nucleus. Fig. 6C) is a view illustrating that approximately 20% of the cells exhibited cytosolic delivery by IgG_polyE-LNP.

Fig. 7A) is a view illustrating that, in HeLa cells to which anti-pAkt1-IgG was delivered by IgG_polyE-LNP, caspase activity increased compared to HeLa cells to which the control antibody hIgG was delivered, indicating that apoptosis was significantly induced. Fig. 7B) is a view illustrating that, in HeLa cells to which anti-pAkt1-IgG was delivered by IgG_polyE-LNP, a significant cell growth inhibitory effect was exhibited compared to HeLa cells to which the control antibody hIgG was delivered.

Fig. 8 is a view illustrating that hIgG-AF488 was able to be delivered to the cytosol in HeLa cells, HT1080 cells, MDA-MB-231 cells, and SW480 cells by IgG_polyE-LNP.

Fig. 9 is a view illustrating that both IgG_polyE-LNP prepared in a t-BuOH lipid solution and IgG_polyE-LNP prepared in a 90% t-BuOH lipid solution were able to deliver antibodies to the cytosol in a similar manner.

Fig. 10a is a view illustrating that a peak top of a particle diameter of lipid nanoparticles is approximately 100 nm.

Fig. 10b is a view illustrating the result of particle diameter measurement of IgG_polyE-LNP determined by nanoparticle tracking analysis (NTA method).

Fig. 11 is a view of each cell immunostained in Example 13.

Fig. 12 is a view of each cell immunostained in Example 13.

Fig. 13 is a view illustrating each particle diameter of hIgG_polyE-LNP in Example 13.

Fig. 14 is a view illustrating each antibody encapsulation efficiency of hIgG_polyE-LNP in Example 13.

Fig. 15 is a view illustrating each value of hIgG_polyE-LNP in Example 14.

Fig. 16 is a view of each cell immunostained in Example 14.

Fig. 17 is a view illustrating formation of a droplet in Example 15.

Fig. 18 is a view of each cell immunostained in Example 16.

Description of Embodiments

A composition used in the present disclosure is a composition comprising a protein, a polymer, and a lipid, in which the polymer has an opposite charge to that of the protein at a certain pH. In other words, the composition comprises a protein, a polymer, and a lipid, wherein the protein and the polymer have opposite charges at a predetermined pH. It should be noted that the numerical ranges that can be used in defining the compositions used in the present disclosure are abbreviated for all the numerical values within the ranges. For example, the numerical range of 1 to 50 includes 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

(Protein)

The protein used in the present disclosure is a protein selected from an antibody and an antibody fragment. An antibody is a protein molecule that specifically binds to an antigen and is composed of immunoglobulin (Ig), which includes types such as IgG, IgA, IgM, IgD, and IgE. An antibody fragment is a fragment of a part of an antibody. The isoelectric point

of an antibody or an antibody fragment is generally about pH 7.5 to 9.5.

When the protein is an antibody or an antibody fragment, when these proteins are applied to a human as a pharmaceuticals or a diagnostic agent, the pharmaceutical composition of the present disclosure can be more stably transported and stored, and moreover, it is easy to administer to a human at the time of use, and thus usefulness is high. The protein may have a sugar chain.

High purity means a pharmaceutical level or a level that can be used in a human pharmaceutical in the case of a medical protein, while for other proteins it means a high level of reagent-grade purity, for example, a threshold of 0.1 mass% or less in the total amount of impurities.

As an antigen to which the antibody or antibody fragment used in the present disclosure specifically binds, a polypeptide and peptide present in a cell including cytoplasm and a nucleus, a gene including RNA (ribonucleic acid) including mRNA, rRNA, tRNA, or the like, DNA (deoxyribonucleic acid), or the like, a lipid including a phospholipid such as cardiolipin, a sugar chain bound to the polypeptide, peptide, gene or lipid, or the like can be suitably illustrated as one non-limiting embodiment. Specific methods for producing the antibody are well known to those skilled in the art, and for example, in the case of a monoclonal antibody, the antibody may be produced by a hybridoma method (Kohler and Milstein, Nature 256:495 (1975)) or a recombinant method (U.S. Patent No. 4,816,567). They may also be isolated from phage antibody libraries (Clackson et al., Nature 352:624-628 (1991); Marks et al., J. Mol. Biol. 222:581-597 (1991)). They may also be isolated from a single B-cell clone (N. Biotechnol. 28(5):253-457 (2011)).

A method for producing a chimeric antibody, a humanized antibody or a human antibody for reducing immunogenicity (Journal of Biomedical Science volume 27, 1 (2020)), a method for engineering an antibody for imparting desired properties and kinetics in vivo, a method for producing an antibody fragment, and a method for linking a toxic substance such as Payload to an antibody (Cell, 185, 15, 2789-2805 (2022)) are known, and antibodies and antibody fragments engineered by these known methods can also be appropriately used in the present disclosure.

It has been shown that antibodies delivered into the cytoplasm by receptor-dependent endocytosis and endosomal escape by cholic acid, or by liposome-mediated endocytosis and endosomal escape by light-sensitive benzoporphyrin are translocated into the nucleus by a nuclear localization signal inserted into the sequence (Mol. Pharmaceutics 2016, 13, 1915-1926 and Mol. Pharmaceutics 2015, 12, 9, 3272-3281). In the present disclosure, by attaching such a nuclear localization signal to the antibody, it becomes possible to translocate the antibody according to the present disclosure, which has been delivered into the cytoplasm, into the nucleus.

(Polymer)

As the polymer used in the present invention, a polymer having a charge that cancels the charge of the protein under the conditions of use with the protein to be delivered into the cell is used. The conditions of use are determined at the time of production, in any of the conditions like in blood, and like in cells. An acidic or basic polymer (anionic polymer, cationic polymer) can be used with reference to the result of isoelectric focusing electrophoresis of the protein to be delivered. As the polymer considered to have a charge, a polymer having a carboxyl group or an amino group is considered. Examples of such a polymer include polyamino acids, polyesters, glycosaminoglycans, and polyamides. As the polymer used in the present disclosure, one having an opposite charge is selected under the measurement conditions of the protein to be delivered into cells. For example, a combination in which the polymer and the protein have opposite charges at a predetermined pH is selected. Whether or not the polymer and the protein have opposite charges at a predetermined pH can be confirmed by measuring pI values of the polymer and the protein. It is possible to determine the pI value of a polymer or a protein using a known method such as measurement of the zeta potential of a polymer solution in water or measurement using isoelectric focusing electrophoresis of the protein.

For proteins used in the present disclosure, Patent Literature 1 and JP 2022-122871 A can be referred to.

As the polyamino acid used in the present disclosure, one having an opposite charge is selected under the measurement conditions of the protein to be delivered into a cell. In order for a polymer to associate with a protein, the isoelectric point of the polymer needs to be different from the isoelectric point of the protein described above, and needs to be higher or lower than the isoelectric point of the protein. For example, since the isoelectric point of an antibody or antibody fragment as an example of a protein is pH 7.5 to 9.5 as described above, the isoelectric point of the polymer is preferably other than this range, for example, pH 7.0 or less or 10.0 or more.

Specific examples of the polymer include cationic or anionic polyamino acids and salts thereof. Examples of the anionic polyamino acid include sodium salt of polyglutamic acid and polyaspartic acid, and examples of the cationic polyamino acid include water-soluble salts (for example, hydrochloride) such as polyarginine, polyhistidine, polylysine, polyornithine, and polycitrulline. In the present disclosure, examples of a more preferable embodiment of the polyamino acid include poly-L-glutamic acid sodium salt and poly-L-aspartic acid sodium salt as anionic polyamino acids, and poly-L-arginine hydrochloride, poly-L-lysine hydrochloride, poly-L-arginine hydrobromide, and poly-L-lysine hydrobromide as cationic polyamino acids. The polyamino acid is a homopolymer or copolymer, or a mixture thereof. It is preferable that polyamino acid has a CH2 group between the carboxyl group or amino group of the main chain and the side chain. The

polymerization degree can be appropriately selected from the range of 20 to 3,000. For example, the polymerization degree can be appropriately selected from the polymerization degree ranges of 20 to 3,000, 20 to 2,500, 20 to 2,000, 20 to 1,500, 20 to 1,000, 20 to 500, 20 to 400, 20 to 300, and 20 to 200. In another non-limitingembodiment, it is possible to appropriately select from a range of polymerization degrees of 40 to 3,000, 40 to 2,500, 40 to 2,000, 40 to 1,500, 40 to 1,000, 40 to 500, 40 to 400, 40 to 300, and 40 to 200. In another non-limitingembodiment, it is possible to appropriately select from a range of polymerization degrees of 60 to 3,000, 60 to 2,500, 60 to 2,000, 60 to 1,500, 60 to 1,000, 60 to 500, 60 to 400, 60 to 300, and 60 to 200. In another non-limitingembodiment, it is possible to appropriately select from a range of polymerization degrees of 80 to 3,000, 80 to 2,500, 80 to 2,000, 80 to 1,500, 80 to 1,000, 80 to 500, 80 to 400, 80 to 300, and 80 to 200. In another non-limitingembodiment, it is possible to appropriately select from a range of polymerization degrees of 100 to 3,000, 100 to 2,500, 100 to 2,000, 100 to 1,500, 100 to 1,000, 100 to 500, 100 to 400, 100 to 300, and 100 to 200. For example, in the case of polyglutamic acid, in another non-limitingembodiment, polyglutamic acid having a polymerization degree corresponding to a molecular weight of 3,000 (polymerization degree 20) to 60,000 (polymerization degree 408) can be suitably used. In another non-limitingembodiment, polyglutamic acid having a polymerization degree corresponding to any one or more molecular weights selected from 3,000 (polymerization degree 20) to 50,000 (polymerization degree 340), 3,000 (polymerization degree 20) to 40,000 (polymerization degree 272), 3,000 (polymerization degree 20) to 20,000 (polymerization degree 136), 3,000 (polymerization degree 20) to 15,000 (polymerization degree 102), 3,000 (polymerization degree 20) to 10,000 (polymerization degree 68), 4,000 (polymerization degree 27) to 50,000 (polymerization degree 340), 4,000 (polymerization degree 27) to 40,000 (polymerization degree 272), 4,000 (polymerization degree 27) to 30,000 (polymerization degree 136), 4,000 (polymerization degree 27) to 20,000 (polymerization degree 136), 4,000 (polymerization degree 27) to 15,000 (polymerization degree 102), 4,000 (polymerization degree 27) to 10,000 (polymerization degree 68), 5,000 (polymerization degree 34) to 50,000 (polymerization degree 340), 5,000 (polymerization degree 34) to 40,000 (polymerization degree 272), 5,000 (polymerization degree 34) to 30,000, 5,000 (polymerization degree 34) to 20,000 (polymerization degree 136), 5,000 (polymerization degree 34) to 15,000 (polymerization degree 102), 5,000 (polymerization degree 34) to 10,000 (polymerization degree 68), 6,000 (polymerization degree 40) to 50,000 (polymerization degree 340), 6,000 (polymerization degree 40) to 40,000 (polymerization degree 272), 6,000 (polymerization degree 40) to 30,000, 6,000 (polymerization degree 40) to 20,000 (polymerization degree 136), 6,000 (polymerization degree 40) to 15,000 (polymerization degree 102), 6,000 (polymerization degree 40) to 10,000 (polymerization degree 68), 7,000 (polymerization degree 48) to 50,000 (polymerization degree 340), 7,000 (polymerization degree 48) to 40,000 (polymerization degree 272), 7,000 (polymerization degree 48) to 20,000 (polymerization degree 136), 7,000 (polymerization degree 48) to 15,000 (polymerization degree 102), 7,000 (polymerization degree 48) to 10,000 (polymerization degree 68), 8,000 (polymerization degree 54) to 50,000 (polymerization degree 340), 8,000 (polymerization degree 54) to 40,000 (polymerization degree 272), 8,000 (polymerization degree 54) to 20,000 (polymerization degree 136), 8,000 (polymerization degree 54) to 15,000 (polymerization degree 102), and 8,000 (polymerization degree 54) to 10,000 (polymerization degree 68) can be suitably used.

For example, antibodies including human immunoglobulins and antibody fragments thereof are generally basic and are positively charged at pH 7.4 of plasma, and therefore, an anionic polymer can be used. As such a polymer, polyglutamic acid or polyaspartic acid can be suitably used as the polyamino acid. As the anionic polymer, glycosaminoglycans such as hyaluronic acid and heparin can also be suitably used. In addition, acidic antibodies and antibody fragments are also pharmaceutically approved as therapeutic antibodies in some cases (Journal of Chromatography B, Volumes 1065-1066, 2017, Pages 119-128). Cationic polymers can be used in compositions of the present disclosure including such antibodies or antibody fragments thereof that are negatively charged at pH 7.4 of plasma. As such a polymer, polyarginine, polylysine, and polyornithine are preferable as the polyamino acid.

The polymer can be selected in consideration of the pKa, pI, size, shape, or the like of the protein to be delivered, and in consideration of the pKa, pI, molecular weight, shape, or the like of the polymer to be selected.

Selecting a suitable polymer can reduce the particle diameter of the lipid nanoparticles.

For polyamino acids used in the present disclosure, Patent Literature 1 can be referred to.

(Droplet)

In the present disclosure, the protein including an antibody, an antibody fragment, or the like stably associates with a polymer having an opposite charge through electrostatic interaction to form droplets. Without wishing to be bound by any particular theory, as an embodiment of association between a protein including an antibody, an antibody fragment, or the like and a polymer, an embodiment in which a protein is electrostatically bound to a polymer having an opposite charge, or an embodiment in which a protein is electrostatically bound to a polymer having an opposite charge and is stably encapsulated in a polymer can be illustrated as anembodiment. A complex of a protein and a polyelectrolyte (PPC; protein-polyelectrolyte complex) (Mashiro Mimura et. al., J. Chem. Phys. 150, 064903 (2019)), which is known as one of the high-density forms of proteins, is also illustrated as a non-limiting embodiment of the droplet of the present disclosure, and the formation of the droplet of the present disclosure can be appropriately confirmed by observation under a microscope using

differential interference contrast. In addition, the diameter of the formed droplet can be measured using a particle diameter distribution meter using a dynamic light scattering method, a nanoparticle tracking analyzer using characteristics of both scattered light and Brownian motion, or the like, in addition to obtaining an approximate value using an eyepiece micrometer under a microscope with a transmission electron microscope. The particle diameter of the droplets of the present disclosure is determined by nanoparticle tracking analysis. Droplets of the present disclosure are also produced in the range of 100 nm to 10 μm, 200 nm to 2 μm, or 300 nm to 1 μm by adjusting production conditions.

The ratio (mass ratio) of the polymer to the protein can be appropriatelydetermined. For example, the ratio can be in the range of 1:100 to 1:10, or even in the range of 1:10 to 1:1. The temperature at the time of mixing can also be appropriatelydetermined, and can be, for example, within the range of 15 to 40°C, or further, within the range of 20°C to 30°C. The pH at the time of mixing may be any pH at which the protein and the polymer can electrostatically interact with each other, and is preferably, for example, a pH between the isoelectric point of the protein and the isoelectric point of the polymer. The formed droplets are separated using a step such as centrifugation, filtration, or depth filtration (for example, Japanese Patent No. 5361738 or the like), and the reconstituted droplets as an aqueous solution with an appropriate protein concentration can be provided to the next encapsulation step.

(Lipid)

As the lipid used in the present disclosure, at least one or more lipids capable of forming micelles in water are used as a mixture (hereinafter, also referred to as a lipid mixture). So-called amphiphilic lipids having both hydrophilic and hydrophobic portions can be used. Known lipids can be used as such lipids, including phospholipids known as constituent components of biological membranes, such as phosphatidylinositol, phosphatidylethanolamine, phosphatidylserine, phosphatidic acid, phosphatidylglycerol, and phosphatidylcholine, or the like. Furthermore, PEG lipids such as DMG-PEG5k or the like, as well as amphoteric lipids used as surfactants such as 3-[(3-cholamidopropyl)dimethylammonio]propanesulfonate (CHAPS), anionic lipids such as sodium cholate, and nonionic lipids such as octyl glucoside, can also be appropriately used. Furthermore, for example, neutral phospholipids such as 1,2-distearoyl-sn-glycero-3-phosphocholine (DSPC), or the like, and PEG lipids such as 1,2-distearoyl-sn-glycero-3-phosphoethanolamine-N-methoxy-polyethylene glycol-2000 (DSPE-mPEG2000) or the like may also be used. These lipids may be a complex in which polyethylene glycol, protein, peptide, and sugar are linked.

In a non-limiting embodiment of the present disclosure, the cationic lipid can comprise 20 mol% to 60 mol%, 20 mol% to 55 mol%, 20 mol% to 50 mol%, 20 mol% to 45 mol%, 20 mol% to 40 mol%, 25 mol% to 55 mol%, 25 mol% to 50 mol%, 25 mol% to 45 mol%, 25 mol% to 40 mol%, 30 mol% to 55 mol%, 30 mol% to 50 mol%, 30 mol% to 45 mol%, 30 mol% to 40 mol%, 35 mol% to 55 mol%, 35 mol% to 50 mol%, 35 mol% to 45 mol%, 35 mol% to 40 mol%, 40 mol% to 55 mol%, 40 mol% to 50 mol%, or 40 mol% to 45 mol%, or 39 mol%, 40 mol%, 41 mol%, 42 mol%, 43 mol%, 44 mol%, 45 mol%, 46 mol%, 47 mol%, 48 mol%, 49 mol%, or 50 mol% (or any percentage or range thereof) of the lipid mixture (excluding PEG lipids) present in the lipid nanoparticles of the present disclosure.

In a non-limiting embodiment of the present disclosure in which the lipid nanoparticles comprise a phospholipid as a lipid mixture, the phospholipid in the lipid mixture (excluding PEG lipids) present in the lipid nanoparticle may comprise 2 mol% to 20 mol%, 2 mol% to 15 mol%, 2 mol% to 12 mol%, 2 mol% to 10 mol%, 2 mol% to 8 mol%, 4 mol% to 15 mol%, 4 mol% to 12 mol%, 4 mol% to 10 mol%, or 4 mol% to 8 mol% (or any percentage or range thereof) of the lipid mixture (excluding PEG lipids) present in the lipid nanoparticle of the present disclosure. In another non-limiting embodiment of the present disclosure, the phospholipid in the lipid mixture (excluding PEG lipids) may comprise 8 mol% to 15 mol%, 8 mol% to 12 mol%, or 8 mol% to 10 mol%, or 5 mol%, 6 mol%, 7 mol%, 8 mol%, 9 mol%, 10 mol%, 11 mol%, or 12 mol% (or any percentage or range thereof) in the lipid mixture (excluding PEG lipids) present in the particles.

In other non-limiting embodiments of the present disclosure in which the lipid nanoparticle comprises cholesterol or a cholesterol derivative as the lipid mixture, the cholesterol or cholesterol derivative in the lipid mixture (excluding PEG lipids) may comprise 20 mol% to 60 mol%, 20 mol% to 55 mol%, 20 mol% to 50 mol%, 20 mol% to 45 mol%, 20 mol% to 40 mol%, 25 mol% to 55 mol%, 25 mol% to 50 mol%, 25 mol% to 45 mol%, 25 mol% to 40 mol%, 30 mol% to 55 mol%, 30 mol% to 50 mol%, 30 mol% to 45 mol%, 30 mol% to 40 mol%, 35 mol% to 55 mol%, 35 mol% to 50 mol%, 35 mol% to 45 mol%, 35 mol% to 40 mol%, 40 mol% to 55 mol%, 40 mol% to 50 mol%, or 40 mol% to 45 mol%, or 39 mol%, 40 mol%, 41 mol%, 42 mol%, 43 mol%, 44 mol%, 45 mol%, 46 mol%, 47 mol%, 48 mol%, 49 mol%, or 50 mol% (or any percentage or range thereof) of the lipid mixture (excluding PEG lipids) present in the lipid nanoparticles of the present disclosure.

In other non-limiting embodiments of the present disclosure, the PEG lipid comprises 0.5 mol% to 5 mol%, 0.5 mol% to 8 mol%, 0.5 mol% to 10 mol%, 1.0 mol% to 5 mol%, 1.0 mol% to 8 mol%, 1.0 mol% to 10 mol%, 1.5 mol% to 5 mol%, 1.5 mol% to 8 mol%, 1.5 mol% to 10 mol%, 2.0 mol% to 5 mol%, 2.0 mol% to 8 mol%, 2.0 mol% to 10 mol%, 0.5 mol%, 1.0 mol%, 1.5 mol%, 5 mol%, 8 mol%, or 10 mol% (or any percentage or range thereof) of the lipid mixture (excluding PEG lipids) present in the lipid nanoparticles of the present disclosure. The percentage of PEG lipid present in the lipid nanoparticles of the present disclosure is the target amount at the time of production, and the actual content of PEG lipid present in the formulation may vary, for example, by ±0.5 mol%.

The composition containing a cationic lipid may comprise 20 mol% to 60 mol% of a cationic lipid, 20 mol% to 60 mol% of cholesterol, and 2 mol% to 20 mol% of a phospholipid with 0.5 mol% to 10 mol% of a PEG lipid. In another non-limiting embodiment of the present disclosure, the composition comprising a cationic lipid is a composition comprising 40 mol% to 55 mol% of a cationic lipid, 40 mol% to 55 mol% of cholesterol, and 8 mol% to 15 mol% of a phospholipid with 1.0 mol% to 5 mol% of a PEG lipid.

In the present disclosure, as the cationic lipid, an intracellular environmentally responsive lipid is also suitably used to increase the efficiency of escape from endosomes after delivery to cell. An intracellular environmentally responsive lipid is a lipid that degrades or changes the structure in response to environmental factors such as reducing conditions and pH in the cell. As the intracellular environmentally responsive lipid, an intracellular environmentally responsive lipid having one or more properties selected from an ionizable property, a reducing environment responsive property, and a self-degradable property can be appropriately used.

Lipids having an ionizable property have no charge in the extracellular environment, but after being taken up into the cell via endocytosis, they become positively charged under the acidic conditions within the endosome and is capable of fusing with the endosomal membrane having a negative charge. Non-limiting examples of such lipids having an ionizable property include a combination of 1,2-dioleoyl-sn-glycero-3-phosphoethanolamine (DOPE) and cholesterol hemisuccinic acid (CHEMS), and a combination of 1,2-dioleoyl-3-dimethylammonium propane (DODAP) and phosphatidylserine (PS), which are appropriately used. 1,2-dilinoleyl-MC3-N,N-dimethyl-3-aminopropane (DLin-MC3-DMA), which has a tertiary amine in the hydrophilic portion of the amphiphilic lipid, is also included as a non-limiting example of such a lipid having an ionizable property.

Lipids having a reducing environment responsive property have no charge in the extracellular environment, but after being taken up into the cell via endocytosis, they become positively charged under the acidic conditions within the endosome and is capable of fusing with the endosomal membrane having a negative charge. Non-limiting examples of such lipids having an ionizable property include a combination of 1,2-dioleoyl-sn-glycero-3-phosphoethanolamine (DOPE) and cholesterol hemisuccinic acid (CHEMS), and a combination of 1,2-dioleoyl-3-dimethylammonium propane (DODAP) and phosphatidylserine (PS), which are appropriately used. 1,2-dilinoleyl-MC3-N,N-dimethyl-3-aminopropane (DLin-MC3-DMA), which has a tertiary amine in the hydrophilic portion of the amphiphilic lipid, is also included as a non-limiting example of such a lipid having an ionizable property.

As a non-limiting embodiment of a lipid having, in addition to the ionizable property and the reducing environment responsive property, the self-degradable property, lipids illustrated in WO 2019-188867, for example, lipids represented by the following Formula (1), can be used.

[Chemical Formula 1]

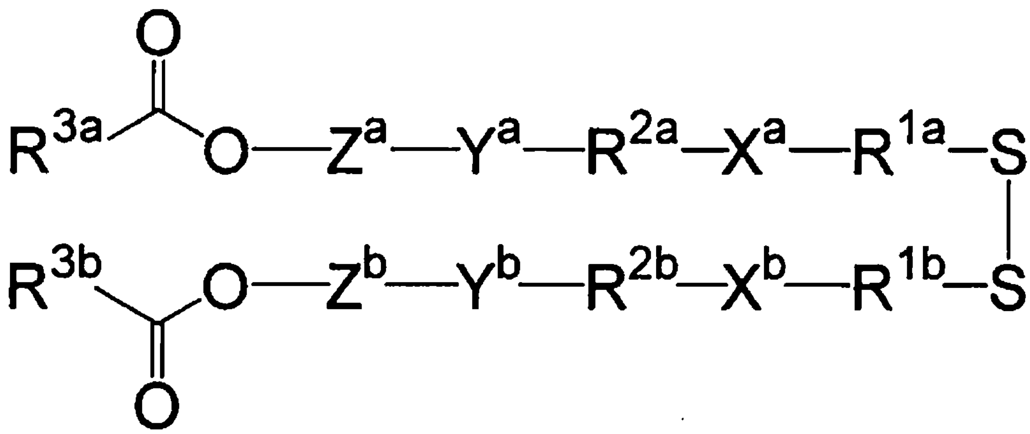

(1)

(In Formula (1),

R1a and R1b each independently represent an alkylene group having 1 to 6 carbon atoms,
Xa and Xb each independently represent a non-cyclic alkyl tertiary amino group having 1 to 6 carbon atoms and one tertiary amino group, or a cyclic alkylene tertiary amino group having 2 to 5 carbon atoms and 1 to 2 tertiary amino groups,
R2a and R2b each independently represent an alkylene group or an oxydialkylene group having 8 or less carbon atoms,
Ya and Yb each independently represent an ester bond, an amide bond, a carbamate bond, an ether bond, or a urea

bond,

Za and Zb each independently represent a divalent group derived from an aromatic compound having 3 to 16 carbon atoms, having at least one aromatic ring, and optionally having a heteroatom, and

R3a and R3b each independently represent a residue derived from a reaction product of a fat-soluble vitamin having a hydroxyl group and succinic anhydride or glutaric anhydride, or a residue derived from a reaction product of a sterol derivative having a hydroxyl group and succinic anhydride or glutaric anhydride, or an aliphatic hydrocarbon group having 12 to 22 carbon atoms.)

More specific examples thereof include O-Ph-P3C1, O-Ph-P4C1, O-Ph-P4C2, O-BnP4C2, E-Ph-P4C2, L-Ph-P4C2, HD-Ph-P4C2, O-Ph-amide-P4C2, and O-Ph-C3M. These lipids respond to pH, redox activity, or the like, and can increase endosomal escape efficiency.

[Chemical Formula 2]

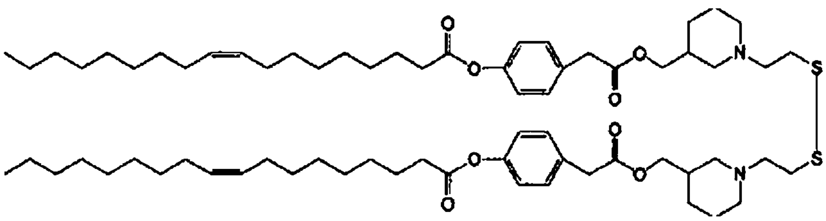

O-Ph-P3C1

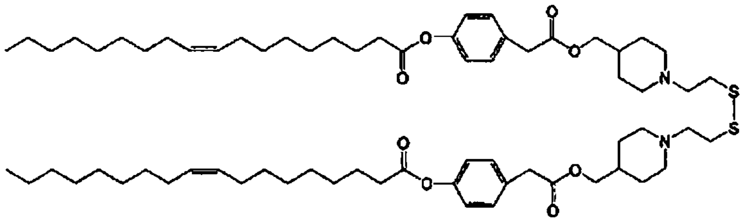

O-Ph-P4C1

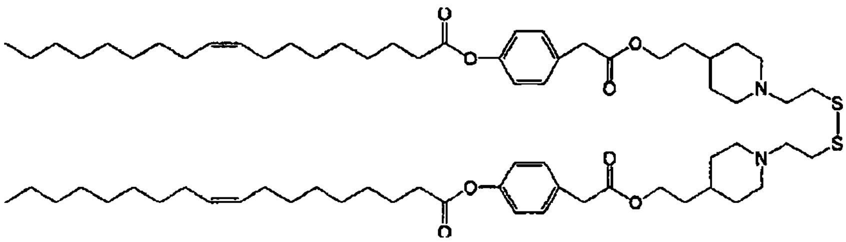

O-Ph-P4C2

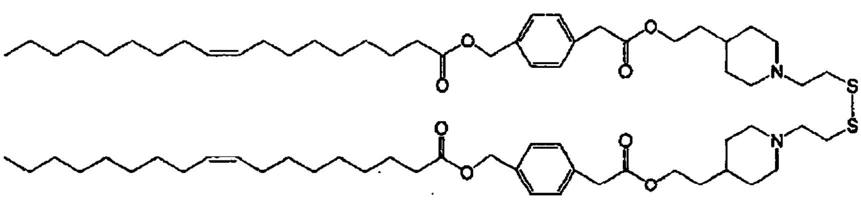

O-Bn-P4C2

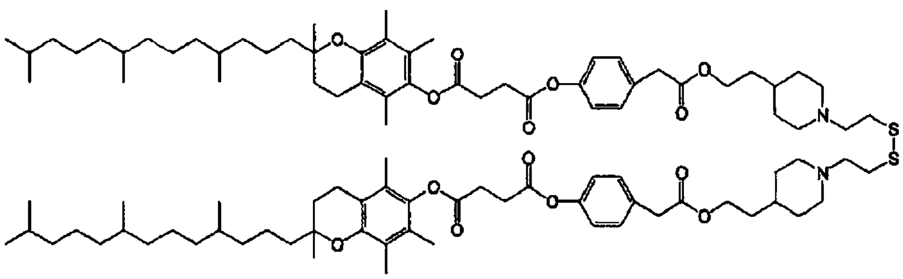

E-Ph-P4C2

[Chemical Formula 3]

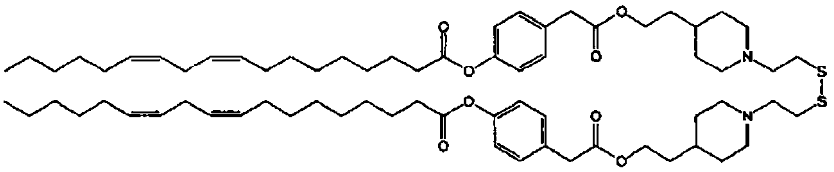

L-Ph-P4C2

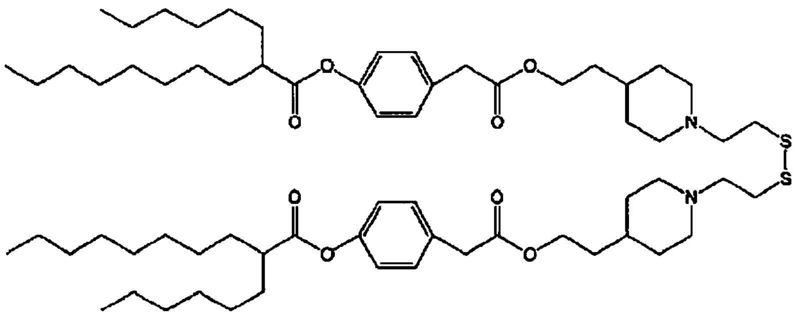

HD-Ph-P4C2

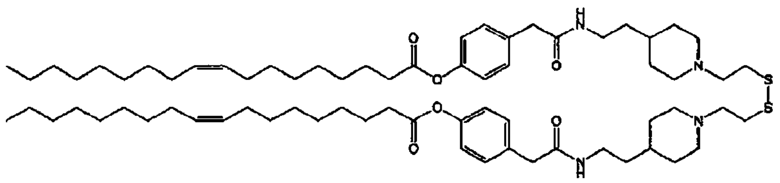

O-Ph-amide-P4C2

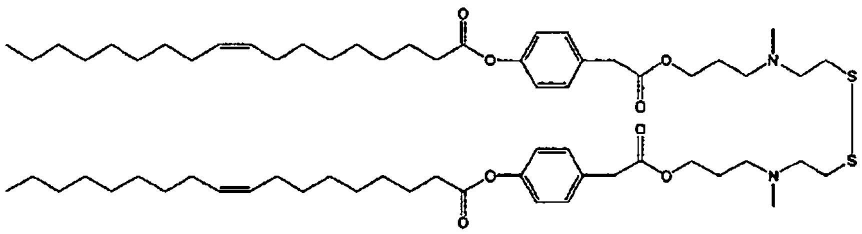

O-Ph-C3M

As the lipid, a compound described in Examples described later, that is, one or more lipids selected from bis [[4-[2-[[4-(oleoyloxy)phenyl]acetoxy]ethyl]piperidino]ethyl] disulfane (SS-OP) as a lipid having an ionizable property, a reducing environment responsive property, and a self-degradable property, 1,2-dioleoyl-sn-glycero-3-phosphoethanolamine (DOPE) as a neutral phospholipid, DMG-PEG5k as a PEG lipid, β-sitosterol as cholesterol, or the like can be suitably used.

The content (protein loading rate) of the antibody in the lipid nanoparticle of the present disclosure can be appropriately prepared within the range of 5 to 95 mol%, 10 to 90 mol%, and 30 to 80 mol%. The lipid mixture of the present disclosure can be prepared by dispersing the cationic lipid of the present disclosure and other constituent components (lipids or the like) in an appropriate solvent or dispersion medium, for example, an aqueous solvent or an alcoholic solvent, and performing an operation of inducing organization as necessary.

(Other Additives)

In producing the lipid nanoparticles of the present disclosure, one or more amphiphilic substances can be used. The amphiphilic substance is selected in consideration of factors such as the stability, uniformity, or the like of the lipid nanoparticles. Examples of amphiphilic substances used in the present disclosure include, but are not limited to: phosphoglycerides; phosphatidylcholine; dipalmitoylphosphatidylcholine (DPPC); dioleoylphosphatidylethanolamine (DOPE); dioleoyloxypropyltrimethylammonium (DOTMA); dioleoylphosphatidylcholine; cholesterol; cholesterol esters; diacylglycerol; diacylglycerol succinate; diphosphatidylglycerol (DPPG); hexadecanol; aliphatic alcohols such as polyethylene glycol (PEG); polyoxyethylene-9-lauryl ether; surface-active fatty acids such as palmitic acid or oleic acid; fatty acids; fatty acid monoglycerides; fatty acid diglycerides; fatty acid amides; sorbitan trioleate (Span (registered trademark) 85) glycocholate; sorbitan monolaurate (Span (registered trademark) 20); polysorbate 20 (Tween (registered trademark) 20); polysorbate 60 (Tween (registered trademark) 60); polysorbate 65 (Tween (registered trademark) 65); polysorbate 80 (Tween (registered trademark) 80); polysorbate 85 (Tween (registered trademark) 85); polyoxyethylene monostearate;

surfactin; poloxamer; sorbitan fatty acid esters such as sorbitan trioleate; lecithin; lysolecithin; phosphatidylserine; phosphatidylinositol; sphingomyelin; phosphatidylethanolamine (cephalin); cardiolipin; phosphatidic acid; cerebroside; dicetyl phosphate; dipalmitoylphosphatidylglycerol; stearylamine; dodecylamine; hexadecylamine; acetyl palmitate; glycerol ricinoleate; hexadecyl stearate; isopropyl myristate; tyloxapol; poly(ethylene glycol) 5000-phosphatidylethanolamine; poly(ethylene glycol) 400-monostearate; phospholipids; synthetic and/or natural detergents with high surfactant activity; deoxycholate; cyclodextrin; chaotropic salts; ion-pairing agents; and combinations thereof. As other additives, substances that contribute to the stability of the lipid nanoparticle membrane can be added. Examples of such a substance include cholesterol, phytosterol, stigmasterol, and β-sitosterol.

(Lipid Nanoparticle)

The lipid nanoparticle can be produced by a production method described later. The lipid nanoparticle has a structure in which a droplet formed by associating a protein including an antibody, an antibody fragment, or the like with a polymer is encapsulated in a lipid. In addition, the protein including an antibody, an antibody fragment, or the like stably associates with a polymer having an opposite charge through electrostatic interaction to form droplets. The embodiment of association between a protein including an antibody, an antibody fragment, or the like and a polymer is not limited to any particular theory, but an embodiment in which a protein is electrostatically bound to a polymer having an opposite charge or an embodiment in which a protein is electrostatically bound to a polymer having an opposite charge and is stably encapsulated in a polymer can be illustrated as an example. The particle diameter of the lipid nanoparticles can be measured using a particle diameter distribution meter using a dynamic light scattering method, a nanoparticle tracking analyzer using both characteristics of scattered light and Brownian motion, or, the like. The particle diameter of the lipid nanoparticles in the present disclosure is determined by nanoparticle tracking analysis and is produced in the range of 10 to 600 nm. Production can be performed even in the range of 50 to 500 nm and 70 to 400 nm by adjusting production conditions. The average particle diameter distribution determined by nanoparticle tracking analysis is 300 nm or less. The peak top is produced at 150 nm or less, and can be produced at 120 nm or less or 100 nm or less by adjusting production conditions. It is said that when the particle diameter is less than 100 nm, it is likely to undergo endocytosis. Furthermore, in tumor cells and the like, it is said that particles with a diameter less than 100 nm tend to accumulate around cancer tissues through the Enhanced Permeability and Retention effect, thereby increasing selectivity.

The content (protein loading rate) of the antibody in the lipid nanoparticle of the present disclosure can be appropriately prepared from the range of 0.1 to 10.0 mol%, 0.2 to 10.0 mol%, 0.3 to 10.0 mol%, 0.4 to 10.0 mol%, 0.5 to 10.0 mol%, 0.1 to 5.0 mol%, 0.2 to 5.0 mol%, 0.3 to 5.0 mol%, 0.4 to 5.0 mol%, 0.5 to 5.0 mol%, 0.1 to 2.0 mol%, 0.2 to 2.0 mol%, 0.3 to 2.0 mol%, 0.4 to 2.0 mol%, and 0.5 to 2.0 mol%.

(Pharmaceutical Composition)

In order to formulate the lipid nanoparticles of the present disclosure as a pharmaceutical composition, it is possible to combine the lipid nanoparticles with various pharmaceutically acceptable additives and a base or carrier for dispersing the active agent(s). Examples of additives include pH adjusters such as arginine, sodium hydroxide, glycine, hydrochloric acid, citric acid, and mixtures thereof. Other additives include local anesthetics (for example, benzyl alcohol), isotonic agents (for example, sodium chloride, mannitol, and sorbitol), adsorption inhibitors (for example, Tween 80), solubility enhancers (for example, cyclodextrins and derivatives thereof), stabilizers (for example, serum albumin), and reducing agents (for example, glutathione). Pharmaceutical compositions containing the lipid nanoparticles of the present disclosure can be dispersed in a base or vehicle, which may include a hydrophilic compound having the ability to disperse the active agent and any desired additives. The base may be selected from a wide range of suitable carriers including, but not limited to, copolymers of polycarboxylic acids or salts thereof, copolymers of carboxylic acid anhydrides (for example, maleic anhydride) with other monomers (for example, methyl (meth)acrylate, acrylic acid, or the like), hydrophilic vinyl polymers such as polyvinyl acetate, polyvinyl alcohol, polyvinylpyrrolidone; cellulose derivatives such as hydroxymethyl cellulose, hydroxypropyl cellulose, or the like; and natural polymers such as chitosan, collagen, sodium alginate, gelatin, hyaluronic acid, non-toxic metal salts thereof, or the like. In many cases, biodegradable polymers such as polylactic acid, poly(lactic acid-glycolic acid) copolymer, polyhydroxybutyric acid, poly(hydroxybutyric acid-glycolic acid) copolymer, or the like, or mixtures thereof are selected as the base or carrier. Instead of the base or the carrier, or in addition to the base or the carrier, a synthetic fatty acid ester such as a polyglycerin fatty acid ester or a sucrose fatty acid ester can also be used as the carrier. Hydrophilic polymers and other carriers can be used alone or in combination and can impart enhanced structural integrity to the carrier by partial crystallization, ionic bonding, crosslinking, or the like. The carrier may be provided in a variety ofembodiments, including fluid or viscous solutions, gels, pastes, powders, microspheres, and films for direct application to nasal mucosa. The use of selected carriers in this context may result in enhanced absorption of the biologically active agent.

The pharmaceutical compositions of the present disclosure can also comprise as carriers pharmaceutically

acceptable substances necessary to approach physiological conditions, such as pH adjusting and buffering agents, tonicity adjusting agents, and wetting agents, for example, sodium acetate, sodium lactate, sodium chloride, potassium chloride, calcium chloride, sorbitan monolaurate, triethanolamine oleate, and mixtures thereof. In the case of solid compositions, conventional non-toxic pharmaceutically acceptable carriers are available, including, for example, pharmaceutical standards mannitol, lactose, starch, magnesium stearate, sodium saccharin, talc, cellulose, glucose, sucrose, magnesium carbonate, or the like.

(Production Method)

Lipid nanoparticles can be produced based on a known method with reference to J. Chem. Phys. 150, 064903 (2019) or the like.

Specifically, first, a protein including an antibody, an antibody fragment, or the like and a polymer are mixed at an appropriate ratio to form a droplet (droplet forming step). The embodiment of association between a protein including an antibody, an antibody fragment, or the like and a polymer, is not limited to any particular theory, but an embodiment in which a protein is electrostatically bound to a polymer having an opposite charge, an embodiment in which a protein is electrostatically bound to a polymer having an opposite charge and is stably encapsulated in a polymer through electrostatic binding, or the like, can be illustrated as an example. Mixing can be performed by operations such as mixing with a liquid mixer or pipetting, stirring with a stirrer or a stirring blade in a mixing container, mixing using a confined impinging jets mixer or a multi-inlet vortex mixer, and contacting on a flow path (that is, the microchannel) of a microfluidic device. The ratio (mass ratio) of polymer to protein can be appropriately determined, and can be, for example, within the range of 100:1 to 10:1, and further within the range of 10:1 to 1:1. The temperature at the time of mixing can also be appropriately determined, and can be, for example, within the range of 15 to 40°C, or within the range of 20°C to 30°C. The pH at the time of mixing may be any pH at which the protein and the polymer can electrostatically interact with each other, and is preferably, for example, a pH between the isoelectric point of the protein and the isoelectric point of the polymer. The formed droplets are separated using a step such as centrifugation, filtration, or depth filtration (for example, Japanese Patent No. 5361738 or the like), and the reconstituted droplets as an aqueous solution with an appropriate protein concentration can be provided to the next encapsulation step.

Next, this droplet and a lipid are mixed to encapsulate the droplet in the lipid (encapsulation step), thereby producing lipid nanoparticles. This mixing can be performed by operations such as mixing with a liquid mixer or pipetting, stirring with a stirrer or a stirring blade in a mixing container, and mixing using a confined impinging jets mixer or a multi-inlet vortex mixer. In addition, operations such as contact on a flow path of a microfluidic device, and other industrial production methods such as an alcohol dilution (precipitation) method, a hydration method, and an emulsification method have also been established. The ratio (mass ratio) of lipid to droplet can be appropriatelydetermined, and can be, for example, within the range of 1:100 to 10:1, and further within the range of 1:10 to 1:1. The temperature at the time of mixing can also be appropriatelydetermined, and can be, for example, within the range of 15 to 40°C, or further, within the range of 20°C to 30°C. The pH at the time of mixing can be, for example, within the range of 5 to 9, and further, can be within the range of 6 to 8. As a solvent that dissolves lipids when producing lipid nanoparticles, alcohols such as ethanol, butanol, and tert-butanol, ethylene glycol, glycerin, polyethylene glycol, or the like can be used, but they should be selected in consideration of the particle formability, the ability to be delivered to cells, toxicity, or the like comprehensively.

(Effects)

The generated nanoparticles are evaluated in vitro and in vivo. In the present specification, the nanoparticles of the present disclosure were observed to be localized in cells including cytosol, nucleus, or the like.

Examples

Hereinafter, the present disclosure will be described more specifically based on Examples and Comparative Examples, but the present disclosure is not limited to the following Examples. Further, in the following Examples, the expression "%" is on a mass basis (mass percent) unless otherwise particularly defined.

[Materials and Methods]

(Reagent)

The pH-responsive lipid ss-cleavable and pH-activated lipid-like material (ssPalm) O-Phe (SS-OP, NOF Corporation), 1,2-dioleoyl-sn-glycero-3-phosphoethanolamine (DOPE, NOF Corporation), β-sitosterol (Sigma Aldrich), and 1,2-dimyristoyl-rac-glycero-3-methylpolyoxyethyleneglycol chain, molecular weight 5000 (DMG-PEG5k, NOF Corporation) were dissolved in chloroform (Fujifilm Wako Pure Chemical Corporation) at a concentration of 10 mM and stored in a freezer at -30°C until use.

As IgG, Normal Human IgG, Whole Molecule, Purified (manufactured by Fujifilm Wako Pure Chemical Corporation) was used. In the present specification, it is referred to as IgG or hIgG.

Polyglutamic acid of the following size was used: molecular weight 3,000 to 15,000 (Sigma).

(Measurement of Particle Diameter)

Regarding the particle diameter, particle diameter, PdI (particle diameter distribution, and zeta potential (ζ-potential) were evaluated using a Zetasizer Nano ZS (manufactured by Malvern). The collected sample was diluted 100 times with PBS, and measurement was performed.

(Encapsulation Rate)

For the encapsulation rate, the prepared LNP was centrifuged at 20,000 g at 4°C for 30 minutes by a tabletop ultracentrifuge, and the protein concentration of the supernatant was measured to calculate the encapsulation efficiency of the protein. That is, IgG_polyE-LNP was adjusted to have an antibody concentration of 2μM with PBS (-) (final samplevolume: 100 μL). This was centrifuged at 4°C and 20,000 × g for 1 hour to precipitate the LNP fraction. 50 μL of the supernatant was collected, the antibody concentration was measured with Nanodrop, and the encapsulation efficiency was calculated back from the following formula. However, $C_{IgG}$ refers to an IgG concentration calculated from the supernatant obtained by centrifuging 2 μM IgG, and $C_{LNP}$ refers to an IgG concentration calculated from the supernatant obtained by centrifuging IgG_polyE-LNP.

Encapsulation efficiency of IgG (%) = $(C_{IgG} - C_{LNP}) / C_{IgG} \times 100$

(HeLa Cell)

HeLa cells obtained from ATCC were cultured in α-MEM containing 10% bovine serum (BS).

(Dialysis)

As a dialysis membrane, a 2-14k MWCO dialysis membrane (Spectrum Laboratories Inc.) was used. After being filled into the dialysis membrane, dialysis was performed by stirring with a Corning stirrer.

(hIgG-AF488)

For Alexa Fluor 488 labeling, 345 μL of PBS(-) was added to 100 μL of 10 mg/mL hIgG (normal human IgG, Fujifilm Wako Pure Chemical Corporation), followed by the addition of 50 μL of 1 M $NaHCO_3$ to make the solution basic. Then, it was mixed with 10 mg/mL Alexa488-SDP ester (Thermo Fisher Scientific) and gently mixed by inversion for 1 hour at 25°C in the dark. Thereafter, the antibody fraction was fractionated with a PD10 column to purify Alexa Fluor 488 labeled hIgG (hIgG-AF488).

(Anti-GFP Antibody)

The anti-GFP antibody was prepared by Expi293TM Expression System (Thermo Fisher Scientific). Expi293FTM cells were prepared to be 3,000,000 cells/mL, and 25 mL was added to a 125 mL Erlenmeyer flask. 25 μg of a plasmid encoding Anti-GFP-IgG (Addgene, Anti-GFP [N86/38.1R]) was mixed with ExpiFectamineTM293 (Thermo Fisher Scientific) and transfected according to the recommended protocol. 20 hours after transfection, 150 μL of ExpiFectamineTM293 Transfectin Enhancer 1 and 1.5 mL of Enhancer 2 were added and incubated for 5 days. The culture solution was transferred to a 50 mL tube, and centrifuged at 3,200 × g at 4°C for 20 minutes, and the supernatant was collected. The supernatant containing the antibody was sterilized by filtration through a 0.22 μm filter. Further, this solution was adsorbed to HiTrap Protein A High Performance (Cytiva), and was eluted from the column with a purification buffer solution (0.1M Glycine HCl pH = 2.7). Fractions containing IgG were collected and dialyzed against PBS (pH 7.4) to obtain an anti-GFP antibody (anti-GFP-IgG). The collected anti-GFP antibody (anti-GFP-IgG) was stored at 4°C. When using an antibody in a citric acid solution (pH 5), substitution with the citric acid solution (pH 5) was performed using a Micro Bio-Spin (registered trademark) Column 30 (BIORAD) .

(hIgG_polyE)

A poly-L-glutamic acid (polyE, molecular weight 3,000 to 15,000) solution dissolved in 10 mM citric acid solution (pH 5) was incubated under the condition of 40°C for 30 minutes.

Thereafter, IgG (all antibodies) dissolved in PBS(-) or citric acid solution (pH 5) was mixed with the above polyE at IgG:polyE = 5:1 (mass ratio) by pipetting 10 times. Thereafter, the mixture was filled into a 12-14k MWCO dialysis membrane (Spectrum Laboratories Inc.), floated in PBS (pH 7.4) at a volume at least 1,000 times greater, and dialysis was performed by stirring with a stirrer.

(Example 1)

(Production of LNP)

Lipids were dissolved in chloroform to prepare 20 mM SS-OP, 10 mM DOPE, 20 mM β-sitosterol, and 10 mM DMG-PEG5k. A composition of SS-OP/DOPE/β-sitosterol/DMG-PEG5k (molar ratio 45/10/45/1.5) was mixed in a flask at room temperature, 1 mL of t-BuOH (Fujifilm Wako Pure Chemical Corporation) was added to dissolve the mixture, and then chloroform was evaporated with a rotary evaporator to obtain a lipid solution. Furthermore, this lipid solution was lyophilized for 18 to 24 hours, and then dissolved with t-BuOH to produce a 10 mM lipid solution. The lipid solution dissolved in t-BuOH and the polyglutamic acid solution (0.75 mg/mL polyglutamic acid solution (polyE, molecular weight 3,000 to 15,000) solution dissolved in a 10 mM citric acid solution (pH 5) were separately incubated for 30 minutes under the condition of 40°C.

hIgG and polyE were mixed 10 times by pipetting such that the ratio was hIgG:polyE = 5:1 (mass ratio) to form hIgG-polyE droplets, and then the lipid solution and the droplet solution incubated at 40°C for 30 minutes were mixed 15 times by pipetting such that the ratio was 1:4 (volume ratio). Thereafter, dialysis was performed against PBS(-) (pH 7.4) in a volume at least 1,000 times greater using a 12-14k MWCO dialysis membrane (Spectrum Laboratories Inc.), and a lipid nanoparticle encapsulating hIgG (hIgG_polyE-LNP) was prepared.

(Comparative Example 1)

(Production of LNP)

An LNP-converted sample (hIgG-LNP) was produced in the same manner as in Production Example 1 except that only hIgG was used without containing polyE.

(Evaluation)

The particle diameter, PdI, and ζ-potential of the hIgG_polyE-LNP prepared in Example 1 and the hIgG-LNP prepared in Comparative Example 1 were evaluated using a Zetasizer Nano ZS (Malvern). The results are shown in Fig. 10a, Fig. 10b, and Table 1. The prepared LNP was centrifuged with a tabletop ultracentrifuge at 4°C and 20,000 g for 30 minutes, and the antibody concentration of the supernatant was measured to calculate the encapsulation efficiency of the antibody. The results are shown in Table 1.

(Evaluation)

The results are shown in Table 1.

[Table 1]

| LNP | Particle diameter (d.nm) | PdI | ξ-potential (mV) | Antibody encapsulation efficiency (%) |
|---|---|---|---|---|
| IgG-LNP | 645 ± 143 | 0.605 ± 0.10 | -6.6 ± 3.1 | 34.3 ± 3.2 |
| IgG_polyE-LNP | 294 ± 42 | 0.392 ± 0.03 | -5.6 ± 0.8 | 33.5 ± 1.8 |

In the measurement of the particle diameter by the NTA method, the above LNP diluted 100 times with PBS was measured using NanoSight NS300. As a result, the particle diameter was 123 ± 2 nm.

(Example 2)

(Production of LNP)

In Example 1, hIgG_polyE-LNP was produced by performing the same operations as those of Example 1 except using a 90% tBuOH aqueous solution instead of tBuOH.

(Effects)

(Example 3) (Localization to HeLa Cells)

(Production of LNP)

In order to examine whether the delivery activity of hIgG to the cytosol is changed by the mixing ratio of the antibody and the lipid, the same operation (as in Example 1) was performed except that hIgG-AF488 was used instead of IgG, and IgG_polyE-LNPs (a) to (e) having mixing ratios of antibody:lipid (a) to (e) were adjusted.
hIgG-AF488:polyE

(a) 1:100
(b) 1:200
(c) 1:300
(d) 1:400
(e) 1:500

The adjusted hIgG-AF488_polyE-LNP was added to and suspended in a serum-containing medium of HeLa cells such that the IgG concentration was 1 μM, and incubated for 18 hours under the conditions of 37°C and 5% $CO_2$, and intracellular localization of hIgG-AF488 was observed. The (b) 1:200 IgG_polyE-LNP delivered hIgG to the cytosol most efficiently (Fig. 1A)). In addition, it was found that, in the case of the (b) 1:200 IgG_polyE-LNP, changes in the time course were observed, and hIgG was delivered to the cytosol 6 hours after addition.

(Example 4)

(Production of Various LNPs)

(Anti-GFP antibody_polyE-LNP)

Anti-GFP antibody_polyE-LNP was produced by performing the same operations as in Example 1, except that an anti-GFP antibody was used instead of hIgG.

(Example 5)

To HeLa cells, (a) hIgG-AF488, (b) hIgG-AF488_PolyE, and (c) hIgG-AF488_PolyE-LNP were added to a serum-containing medium to be 1 μM, and incubated for 18 hours under the conditions of 37°C and 5% CO2, and intracellular localization of IgG was observed (Fig. 2A).

In the cells to which (a) hIgG-AF488 and (b) hIgG-AF488_polyE were added, only a dotted signal derived from hIgG-AF488 was observed, and it was confirmed that the signal did not translocate to the cytosol. Meanwhile, in the cells to which (c) hIgG_polyE-LNP sample was added, it was confirmed that signals of IgG_polyE-LNP were diffused throughout the cytosol.

To confirm that the observed signal was due to delivery of IgG, after treatment, cells were fixed with paraformaldehyde and immunostained using Alexa fluor 594 labeled anti-hIgG antibody (anti-hIgG-IgG labeled with Alexa Fulor 594) to observe cellular localization of hIgG (Fig. 2B). It was found that an Alexa Fluor 488 signal present in the cytosol is due to the delivery of IgG molecules, as an IgG-AF488 signal in the cytosol was colocalized with the signal of the anti-hIgG secondary antibody.

(Example 6)

In order to demonstrate the usefulness of adding polyE, LNP (IgG-LNP) (Reference Example 1) obtained by mixing hIgG not containing polyE and lipid was prepared, and a comparative test with hIgG_polyE-LNP (Example 1) was

performed. In order to observe intracellular localization, hIgG-AF488 was used as hIgG.

HeLa cells were suspended in serum-containing medium to which hIgG-LNP and hIgG_polyE-LNP were added at an IgG concentration of 1 μM, and incubated at 37°C with 5 % CO2 for 18 hours. It was confirmed that, when observing the intracellular localization of hIgG-AF488, the fluorescence signal observed in the cytosol of IgG_polyE-LNP is clearly higher than that of IgG-LNP (Fig. 3A). When the amount of antibody translocated into cells by each LNP is quantified by flow cytometry, the cellular uptake amount of hIgG_polyE-LNP was increased approximately two times that of hIgG-LNP (Fig. 3B).

(Example 7)

LgBiT and HiBiT associate with each other to reconstitute nanoLuciferase, and when luciferin is added, luminescence is exhibited. Using this technology, the cytosol translocation amounts of hIgG_polyE-LNP (Production Example 1) and hIgG-LNP (Production Example 2) were compared.

To HeLa cells (HeLa-LgBiT) that constitutively express LgBiT, hIgG_polyE-LNP or IgG-LNP encapsulating hIgG-HiBiT, to which a HiBiT sequence genetically engineered to the C-terminus of an anti-GFP antibody (anti-GFP-IgG) (Addgene; Anti-GFP [N86/38.1R], Plasmid #114492) wasattached, was suspended in a serum-containing medium at an IgG concentration of 0.5 μM and administered for 18 hours. After incubation, the cells were washed 3 times with PBS (pH 7.4) and collected by trypsin treatment, then mixed with a NanoGlo (registered trademark) Live Cell Assay reagent (Promega), and the amount of luminescence was measured. As a result, in the cells to which IgG_polyE-LNP was added, luminescence of 5 times or more was shown as compared with that of IgG-LNP (Fig. 4).

(Example 8)

Next, it was examined whether IgG that targets a protein present in the cytosol can recognize the protein molecule upon being delivered by LNP.

HRas is a member of Ras protein family and is a protein that localizes to the cell membrane. By using HeLa cells expressing HRas-GFP, in which GFP is fused to HRas, GFP was localized to the cell membrane. Anti-GFP-IgG labeled with Alexa Fluor 594 (Addgene; Anti-GFP [N86/38.1R], Plasmid #114492) (anti-GFP antibody-AF 594) was produced, formulated into LNP, suspended in a serum-containing medium to have an IgG concentration of 1μM in HRas-GFP-expressing HeLa cells, and then administered for 18 hours to observe intracellular localization of IgG. Colocalization of fluorescence signals of GFP and anti-GFP-IgG-AF594 was confirmed in the vicinity of the cell membrane in which HRas-GFP existed (Fig. 5A).

Delivery of an anti-GFP antibody without a fluorescent label to the cytosol was also examined. Unlabeled anti-GFP antibody was formulated into LNP, suspended in serum-containing medium containing the HRas-GFP-expressing HeLa cells such that the IgG concentration was 1 μM, and incubated for 18 hours. Thereafter, fixation with paraformaldehyde and immunostaining using an Alexa fluor 568 labeled secondary antibody (ThermoFisher Scientific) were performed, and intracellular localization of IgG was observed. Similarly to the fluorescently labeled antibody, it was confirmed that the fluorescence signal of HRas-GFP and the fluorescence signal of Alexa fluor 568 of the secondary antibody were colocalized (Fig. 5B). From the above results, it was found that both the fluorescently labeled antibody and the non-fluorescently labeled antibody can be delivered to the cytosol by IgG_polyE-LNP.

(Example 9)

Next, in order to introduce an antibody that recognizes an endogenous protein, anti-NPC-IgG (Mab414, Cat#: 902902, BioLegend), an antibody against nuclear pore complexes (NPC), was introduced. hIgG-AF488_polyE-LNP or hIgG-AF488_polyE sample was suspended in a serum-containing medium to have an IgG concentration of 1μM, and administered to HeLa cells, for 18 hours. Thereafter, immunostaining was performed to observe intracellular localization of IgG. In the cells to which hIgG-AF488_polyE was added, only a dotted signal was observed, but in the cells to which hIgG-AF488_polyE-LNP was added, an IgG signal was observed in a ring shape around the nucleus (yellow arrow heads) (Fig. 6A). In addition, when a line plot was produced to cross the nucleus, when hIgG-AF488_polyE droplet was added, an antibody signal (magenta color) was confirmed at a position away from the nucleus signal (cyan color), whereas when hIgG-AF488_polyE-LNP treatment was applied, antibody signals were detected to be adjacent to both ends of the nucleus signal (Fig. 6B). Furthermore, about 400 cells were observed in each sample, and the number of cells in which aa ring-shaped antibody signals was observed around the nucleus was counted, and the percentage was calculated. As a result, it was confirmed that the anti-NPC antibody was able to be delivered to the nuclear pore in approximately 20% of the cells by IgG_polyE-LNP treatment, and fluorescence imaging was able to be performed (Fig. 6C).

(Example 10)

Delivery of IgG that exerts cellular functions by inhibiting not only the activity of recognizing intracellular proteins but also the activity of the recognized proteins was performed. Akt suppresses the activity of proteins that induce apoptosis in cancer cells. It has been reported that apoptosis of cells can be induced by introducing IgG (anti-pAKT1-IgG) (Cat#: 700392, Invitrogen) that recognizes phosphorylated Akt (pAkt), an active form of Akt, and inhibits the function thereof, into HeLa cells. Therefore, it was examined whether anti-pAkt1-IgG can be delivered to the cytosol by LNP to induce apoptosis.

IgG_polyE-LNPs encapsulating hIgG as a control IgG and anti-pAkt1-IgG were each suspended in serum-containing medium at an hIgG concentration of 1 μM and administered to HeLa cells for 24 hours. Thereafter, Caspase-Glo (registered trademark) 3/7 Assay reagent (Promega) was added, and the amount of luminescence was quantified after incubation at room temperature for 1 hour. As a result, it was confirmed that apoptosis was significantly induced in the group to which the anti-pAkt1 antibody (anti-pAkt1-IgG) was delivered as compared with hIgG (Fig. 7A). In addition, when the cell viability was examined under the same experimental conditions by WST-8 assay, a significant cell proliferation inhibitory effect was obtained in the group to which LNP for delivering an anti-pAkt1 antibody (anti-pAkt1-IgG) was added (Fig. 7B). From the above results, it was demonstrated that hIgG_polyE-LNP can deliver IgG capable of inhibiting the activity of the protein into the cytosol, and can control the cell function.

(Example 11)

The versatility of IgG_polyE-LNP was examined using HT1080 cells, MDA-MB-231 cells, and SW480 cells in addition to HeLa cells. IgG_polyE-LNPs encapsulating hIgG-AF488 were each suspended in the serum-containing medium at an IgG concentration of 1 μM and administered to the above cells, followed by incubation for 24 hours. Observation with a confocal microscope revealed that in HT1080 cells, hIgG-Alexa488 was efficiently delivered to the cytosol (Fig. 8). Although MDA-MB-231 and SW480 cells were not highly efficient, diffusion into the cytosol was confirmed (Fig. 8). Therefore, it was demonstrated that hIgG_polyE-LNP can be delivered to cytosol also in cancer cells other than HeLa cells.

(Example 12)

Lipids after lyophilization were dissolved in 90% t-BuOH to examine the solvent in the lipid solution. The lipid solution and a 0.75 mg/mL poly-L-glutamic acid (polyE, molecular weight 3,000 to 15,000) solution dissolved in a 10 mM citric acid solution (pH 5) were separately incubated for 30 minutes under the condition of 40°C. Thereafter, 3 μL of hIgG (10 mg/mL) was added to 8 μL of polyE [IgG:polyE = 5:1 (mass ratio)], and 29.8 μL of a citric acid solution was added and mixed by pipetting 10 times to form hIgG-polyE droplets. The lipid solution prepared to 4mM with 90% t-BuOH and incubated at 40°C for 30 minutes and the droplet solution were mixed 15 times by pipetting to be 1:4 (volume ratio). Thereafter, dialysis was performed against PBS(-) in a volume at least 1,000 times greater using a 12-14k MWCO dialysis membrane (Spectrum Laboratories Inc.), and a lipid nanoparticle encapsulating IgG (hIgG_polyE-LNP) was prepared. These were added to HeLa cells, and after 18 hours, the cells were fixed and permeabilized. Immunostaining was performed with Alexa488 labeled anti-human IgG antibody, and the cells were observed with a confocal microscope. As a result, it was found that hIgG_polyE-LNP prepared with a 90% t-BuOH lipid solution was also able to deliver the antibody to the cytosol similarly to hIgG_polyE-LNP prepared with a t-BuOH lipid solution (Fig. 9).

(Microfluidic Device: Example 13)

Preparation of the lipid nanoparticle (hIgG_polyE-LNP) was examined using a microfluidic device.

Human IgG (10 mg/mL) and polyE (0.75 mg/mL) were dissolved in 10 mM MES buffer (pH 5) to be 5:1 (w/w), and droplets were formed and filled in a 1 mL syringe (Terumo).

An 8 mM or 16 mM lipid solution prepared with t-BuOH to be 1.5 mol% PEG5k in a mixed liquid of SS-OP:DOPE:β-sitosterol = 45:10:45 was filled into another 1 mL syringe (Terumo). These were attached in a syringe pump (kd scientific) and mixed at each flow rate, and then the mixed liquid was dialyzed overnight with PBS. Conditions of each sample are shown in the following table.

[Table 2]

| Sample name | Flow rate (μL/min) | Lipid/droplet (v/v) | Lipid conc. (Amount of lipid) |
|---|---|---|---|
| 100 - 4 - 8 | 100 | 1 : 4 | 8 mM |
| 100-8-8 | 100 | 1 : 8 | |
| 500 - 4 - 8 | 500 | 1 : 4 | |
| 500 - 8 - 8 | 500 | 1 : 8 | |
| 100 - 4 - 16 | 100 | 1 : 4 | 16 mM |
| 100 - 8 - 16 | 100 | 1 : 8 | |
| 500 - 4 - 16 | 500 | 1 : 4 | |
| 500 - 8 - 16 | 500 | 1 : 8 | |

HeLa cells were treated with a serum-containing medium containing these lipid nanoparticles (hIgG_polyE-LNP) for 18 hours, and the cells were observed by immunostaining. The results are shown in Figs. 11 and 12. Delivery into cytoplasm was confirmed in both conditions, but the lipid nanoparticle (hIgG_polyE-LNP) produced under the condition of lipid concentration of 8 mM and flow rate of 100 μL/min had the highest delivery efficiency. In addition, the lipid nanoparticle (hIgG_polyE-LNP) prepared with a lipid to droplet flow rate ratio of 1:4 had the highest delivery efficiency.

When the particle diameter of the produced lipid nanoparticle (hIgG_polyE-LNP) was measured (Fig. 13) and the antibody encapsulation efficiency (Fig. 14) was calculated, it was observed that the encapsulation efficiency of the lipid nanoparticle (hIgG_polyE-LNP) produced under the condition of a lipid concentration of 8 mM and a flow rate of 100 μL/min or the lipid nanoparticle (hIgG_polyE-LNP) produced under the condition of a flow rate ratio of lipid to droplet of 1:4 was high, and the particle diameter tended to increase. Meanwhile, the particle diameter of the lipid nanoparticle (hIgG_polyE-LNP) produced under the condition of a flow rate of 500 μL/min tended to be small, and the encapsulation efficiency tended to be low.

It was demonstrated that it was possible to produce the lipid nanoparticle (hIgG_polyE-LNP) exhibiting desired characteristics by changing the mixing conditions of the microfluidic device as described above.

(Size of PolyE: Example 14)

Delivery efficiency into cytoplasm of the lipid nanoparticle (hIgG_polyE-LNP) prepared using droplets containing PolyE having different molecular weights and antibody was confirmed. 4 μL of 10 mg/mL IgG was mixed with 8 μL of each polyglutamic acid solution having a molecular weight of 3-15k, 39k, or 2.6k of 0.375 mg/mL, 0.75 mg/mL, 1.5 mg/mL, or 3.0 mg/mL, then the mixture was prepared (filled up) to 40 μL with a 10 mM citrate buffer solution (pH 5) and then suspended, 10 μL of an 8 mM lipid solution was further added, and the mixture was dialyzed overnight in PBS to produce the lipid nanoparticle (hIgG_polyE-LNP).

HeLa cells were treated at 37°C for 18 hours with lipid nanoparticle (hIgG_polyE-LNP) prepared (filled up) by adding a serum-containing medium to the dialysate and adjusting the volume to 200 μL, and then the cells washed with PBS were fixed with 4% formaldehyde and subjected to membrane permeation treatment with 0.1% Triton X-100. Immunostaining with anti-Human IgG-Alexa488 suggested that the intracellular delivery efficiency of lipid nanoparticles (hIgG_polyE-LNP) prepared using droplets with 2.6k polyglutamic acid was lower in cytosolic delivery efficiency compared to those prepared using droplets with 3-15k or 39k polyglutamic acid (Figs. 15 and 16).

As described above, it was demonstrated that it is possible to produce the lipid nanoparticle (hIgG_polyE-LNP) exhibiting desired characteristics by examining the polymerization degree of the polymer.

(Hyaluronic Acid: Example 15)

Delivery efficiency into cytoplasm of the lipid nanoparticle (hIgG_polyE-LNP) prepared using droplets containing an anionic polymer other than PolyE, specifically, hyaluronic acid having a molecular weight of approximately 1,000,000 (polymerization degree estimation: approximately 2,500) and an antibody was confirmed. When 3 μL of 10 mg/mL IgG was mixed with 8 μL of each of 0.09375 mg/mL, 0.1875 mg/mL, 0.375 mg/mL, 0.75 mg/mL and 1.5 mg/mL hyaluronic acid solutions, formation of droplets was confirmed under a microscope in a mixed liquid prepared such that hyaluronic acid:IgG (w/w) was 1:40 to 1:5 (Fig. 17).

The mixed liquid in which the droplets were formed was prepared (filled up) to 40 μL with a 10 mM citrate buffer solution (pH 5) and then suspended, 10 μL of an 8 mM lipid solution was further added, and the mixture was dialyzed overnight with PBS to produce the lipid nanoparticle (hIgG_polyE-LNP).

HeLa cells were treated at 37°C for 18 hours with lipid nanoparticle (hIgG_polyE-LNP) prepared (filled up) by adding a serum-containing medium to the dialysate and adjusting the volume to 200 μL, and then the cells washed with PBS were fixed with 4% formaldehyde and subjected to membrane permeation treatment with 0.1% Triton X-100. Immunostaining with anti-Human IgG-Alexa488 confirmed that IgG contained in the lipid nanoparticle (hIgG_polyE-LNP) was delivered to the cytosol (Fig. 18).

It was demonstrated that by using an anionic polymer other than PolyE as described above, the formation of droplets containing the anionic polymer and the antibody under a microscope can be confirmed. Furthermore, it was also demonstrated that the antibody contained in the lipid nanoparticle (hIgG_polyE-LNP) prepared using the droplets formed in this manner was efficiently delivered into cells.

(Example 16)

A gene in which a nuclear localization signal sequence (PAAKRVKLD) is added to the C-terminal of an antibody capable of binding to a target molecule present in the nucleus is expressed to prepare an intranuclear molecule-specific antibody (IgG-NLS). 3 μL of 10 mg/mL IgG-NLS and 8 μL of a polyglutamic acid solution at each concentration (0.375 mg/mL, 0.75 mg/mL, 1.5 mg/mL, or 3.0 mg/mL concentration) are mixed, then the mixture is prepared (filled up) into 40 μL with a 10 mM citrate buffer solution (pH5), then suspended, 10 μL of an 8 mM lipid solution is further added, and the mixture is dialyzed overnight in PBS to produce the lipid nanoparticle (IgG-NLS_polyE-LNP).

HeLa cells were treated at 37°C for 18 hours with the lipid nanoparticle (IgG-NLS_polyE-LNP) prepared (filled up) by adding a serum-containing medium to the dialysate and adjusting the volume to 200 μL, and then the cells washed with PBS were fixed with 4% formaldehyde and subjected to membrane permeation treatment with 0.1% Triton X-100. Immunostaining is performed using anti-Human IgG-Alexa488, and the localization of IgG-NLS is observed by a confocal microscope.

Industrial Applicability

By using a polymer having an opposite charge to that of a charged protein to form a lipid nanoparticle, it is possible to produce smaller lipid nanoparticles, which are taken up by cells and delivered into the cells including the cytosol and the nucleus. By utilizing this drug delivery system, it is possible to introduce antibodies, which had been difficult to deliver so far, into cells, and it is possible to broaden the range of therapeutic methods.

## Claims

**1.** A lipid nanoparticle comprising a composition comprising a protein selected from an antibody and an antibody fragment, a polymer, and a lipid, wherein the protein and the polymer have opposite charges at a predetermined pH.

**2.** The lipid nanoparticle according to claim 1,
wherein the protein is encapsulated in the lipid nanoparticle.

**3.** The lipid nanoparticle according to claim 1,
wherein a peak top of a particle diameter distribution is at 80 nm to 120 nm when a particle diameter of the lipid nanoparticle is measured.

**4.** The lipid nanoparticle according to claim 1,
wherein the polymer is an anionic or cationic polymer.

**5.** The lipid nanoparticle according to claim 4,
wherein the cationic polymer is at least one selected from the group consisting of polylysine, polyarginine, polyhistidine, and water-soluble salts thereof.

**6.** The lipid nanoparticle according to claim 4,
wherein the anionic polymer is at least one polyamino acid selected from the group consisting of polyglutamic acid and polyaspartic acid, or at least one glycosaminoglycan selected from the group consisting of hyaluronic acid and pullulan.

**7.** The lipid nanoparticle according to claim 4,
wherein the cationic or anionic polymer has a molecular weight of 0.5 kDa to 1,000 kDa.

**8.** The lipid nanoparticle according to claim 1, wherein the lipid is a cationic lipid.

**9.** The lipid nanoparticle according to any one of claims 1 to 8, further comprising one or more lipids selected from the group consisting of a neutral phospholipid, a PEG lipid, and cholesterol.

**10.** A pharmaceutical composition comprising a composition comprising a protein selected from an antibody and an antibody fragment, a polymer, and a lipid, wherein the protein and the polymer have opposite charges at a predetermined pH.

**11.** A method for producing a lipid nanoparticle comprising:

a droplet forming step of mixing a protein selected from the antibody and the antibody fragment with the polymer to form a droplet; and
an encapsulation step of mixing the droplet and the lipid to encapsulate the droplet with the lipid.

[FIG. 1]

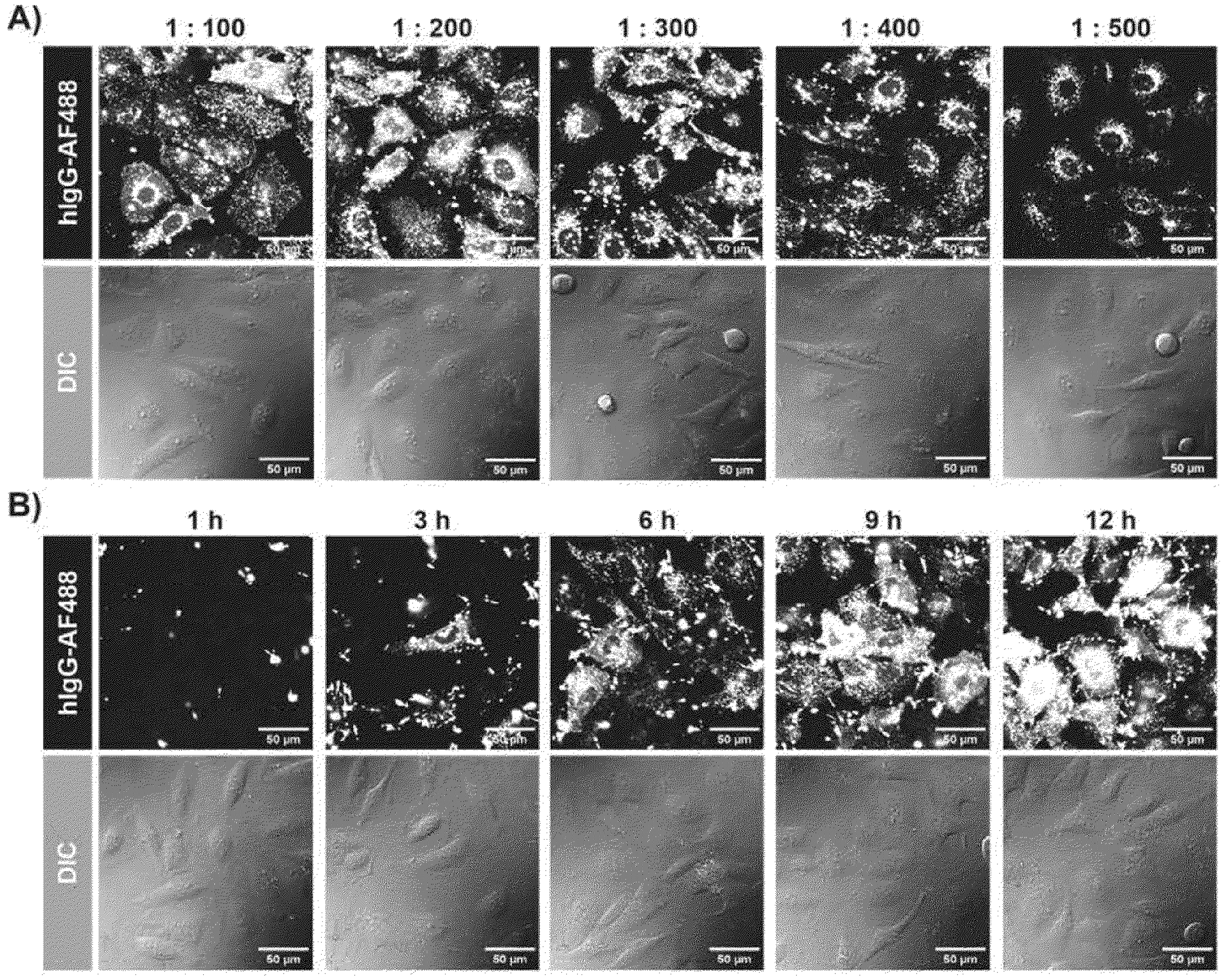
A)
1 : 100
1 : 200
1 : 300
1 : 400
1 : 500
hIgG-AF488
DIC
50 μm
B)
1 h
3 h
6 h
9 h
12 h
hIgG-AF488
DIC
50 μm

[FIG. 2]

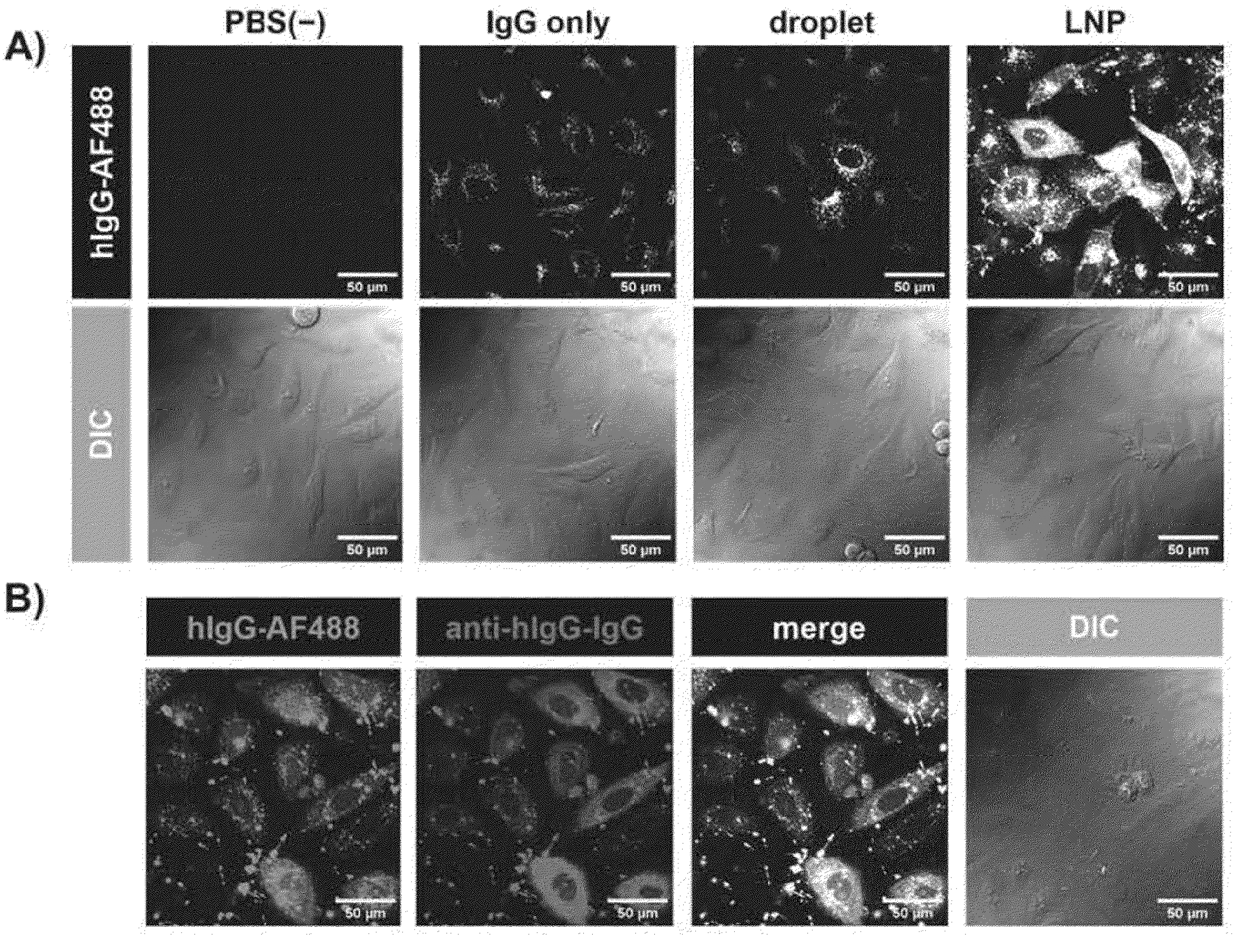
A)
PBS(−)
IgG only
droplet
LNP
hIgG-AF488
DIC
50 μm
B)
hIgG-AF488
anti-hIgG-IgG
merge
DIC
50 μm

[FIG. 3]

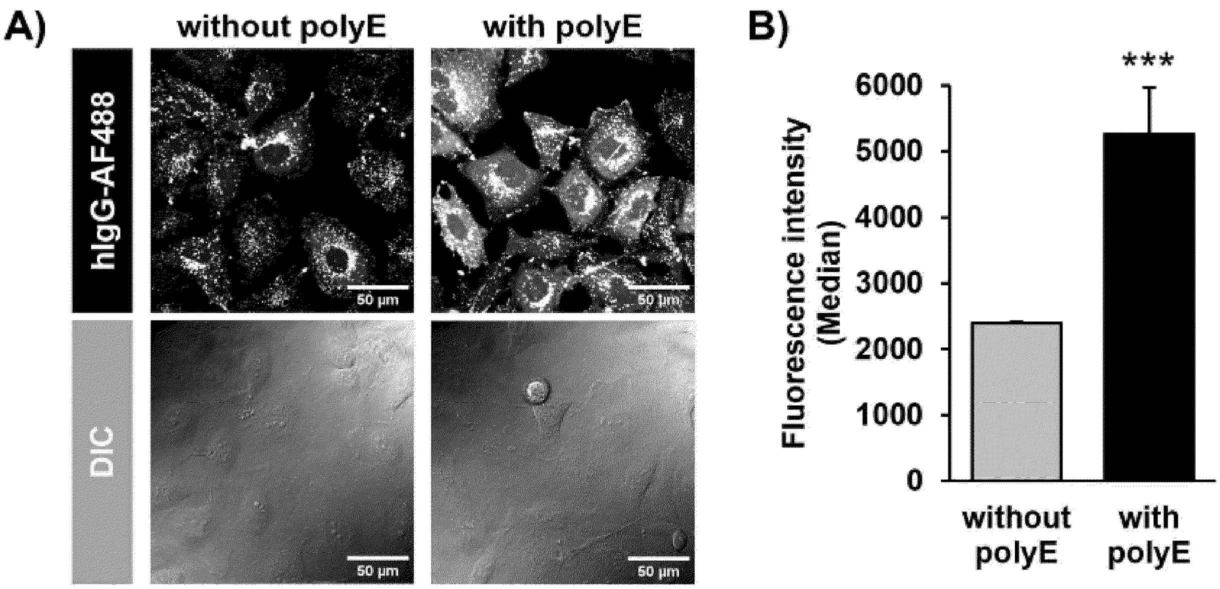
A)
without polyE
with polyE
hIgG-AF488
DIC
50 µm
B)
***
Fluorescence intensity (Median)
6000
5000
4000
3000
2000
1000
0
without polyE
with polyE

[FIG. 4]

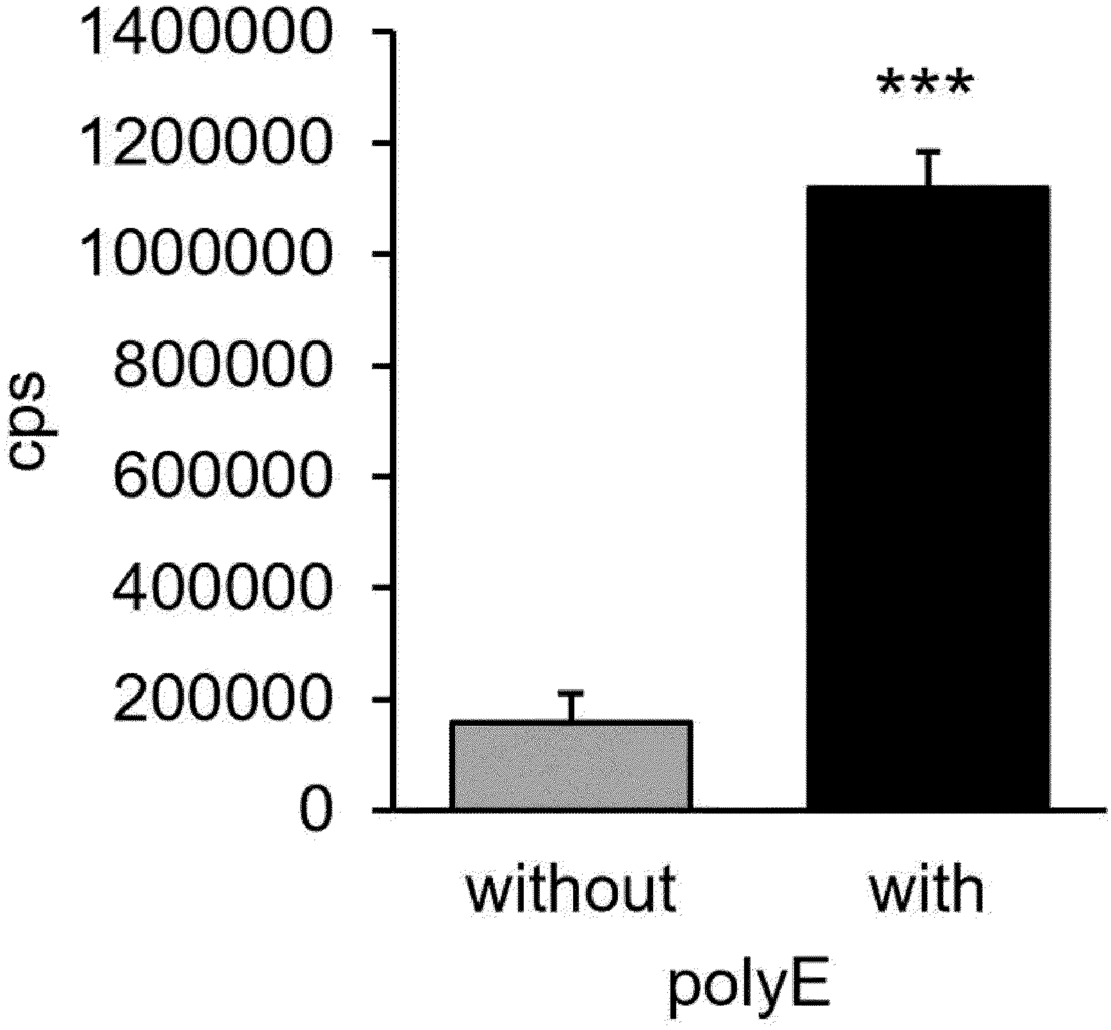
1400000
1200000
1000000
800000
600000
400000
200000
0
cps
***
without
with
polyE

[FIG. 5]

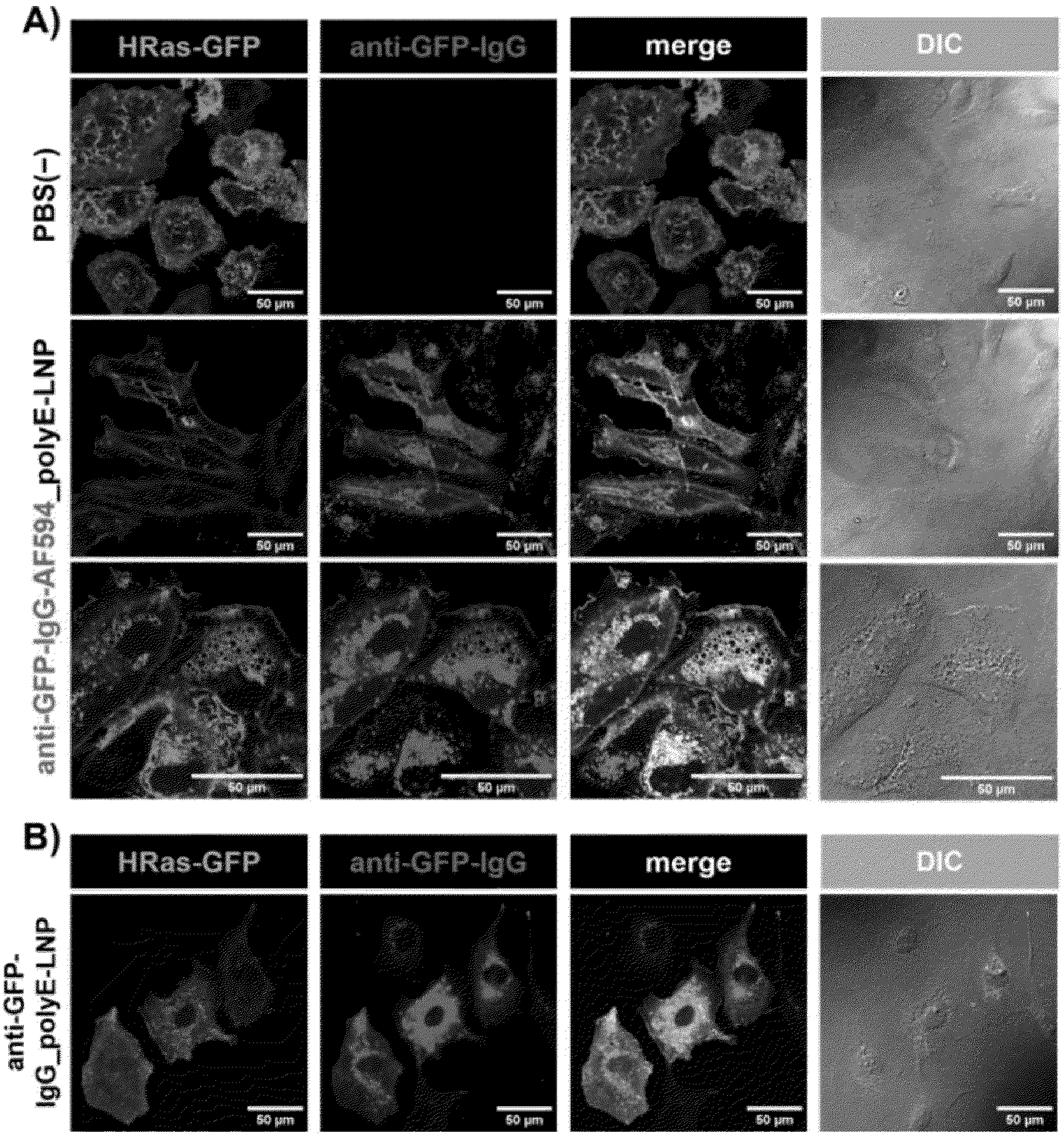
A)
HRas-GFP
anti-GFP-IgG
merge
DIC
PBS(−)
anti-GFP-IgG-AF594_polyE-LNP
50 μm
B)
HRas-GFP
anti-GFP-IgG
merge
DIC
anti-GFP-IgG_polyE-LNP
50 μm

[FIG. 6]

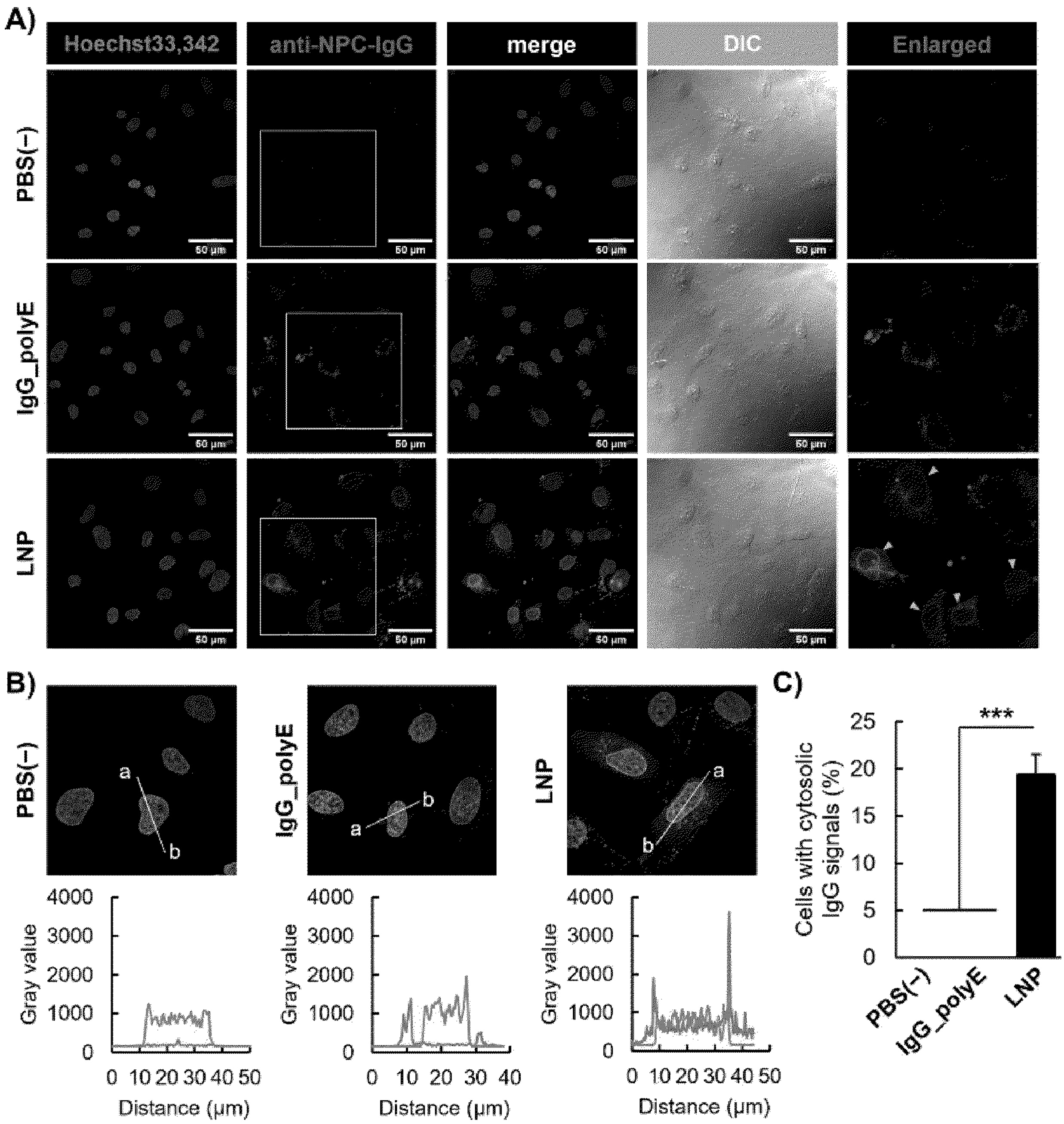
A)
Hoechst33,342
anti-NPC-IgG
merge
DIC
Enlarged
PBS(−)
IgG_polyE
LNP
50 μm
B)
PBS(−)
IgG_polyE
LNP
a
b
Gray value
Distance (μm)
C)
Cells with cytosolic IgG signals (%)
***
PBS(−)
IgG_polyE
LNP

[FIG. 7]

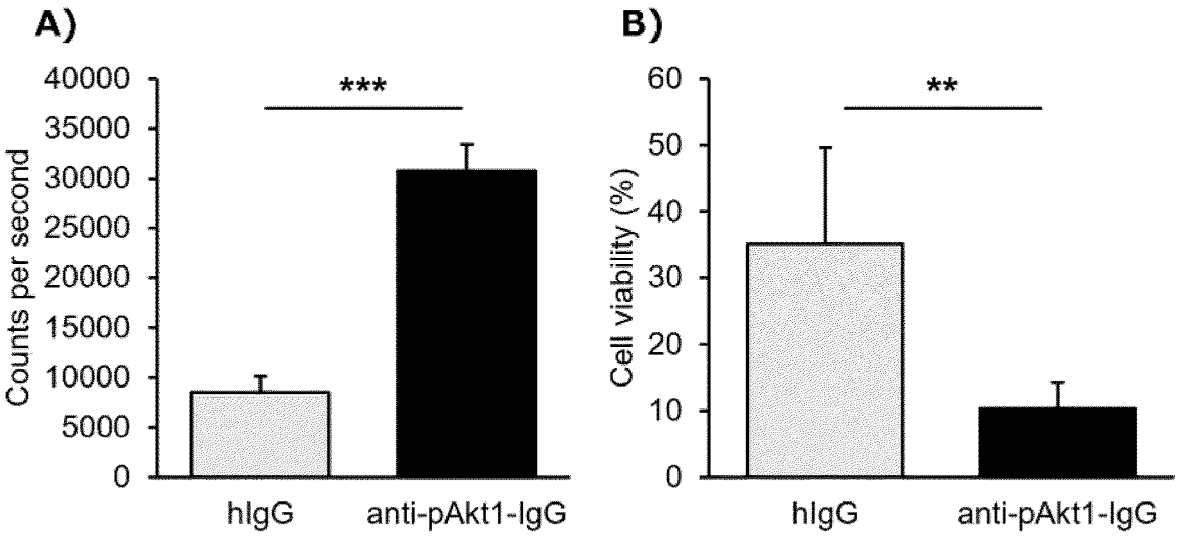
A)
40000
35000
30000
25000
20000
15000
10000
5000
0
Counts per second
***
hIgG
anti-pAkt1-IgG
B)
60
50
40
30
20
10
0
Cell viability (%)
**
hIgG
anti-pAkt1-IgG

[FIG. 8]

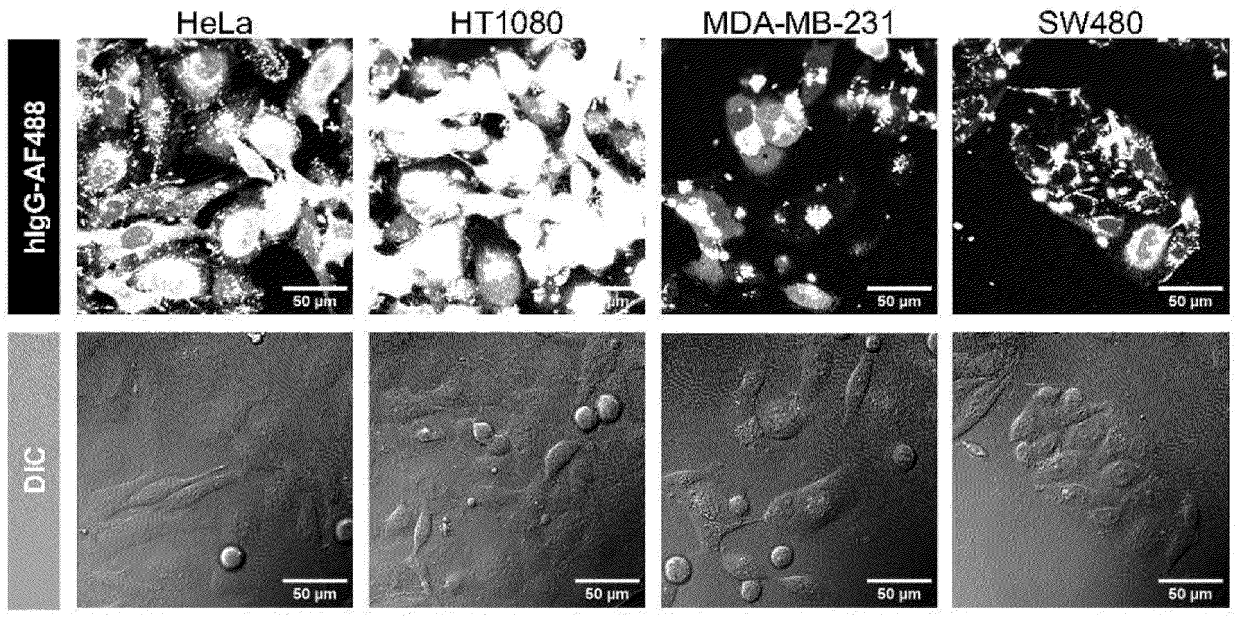

[FIG. 9]

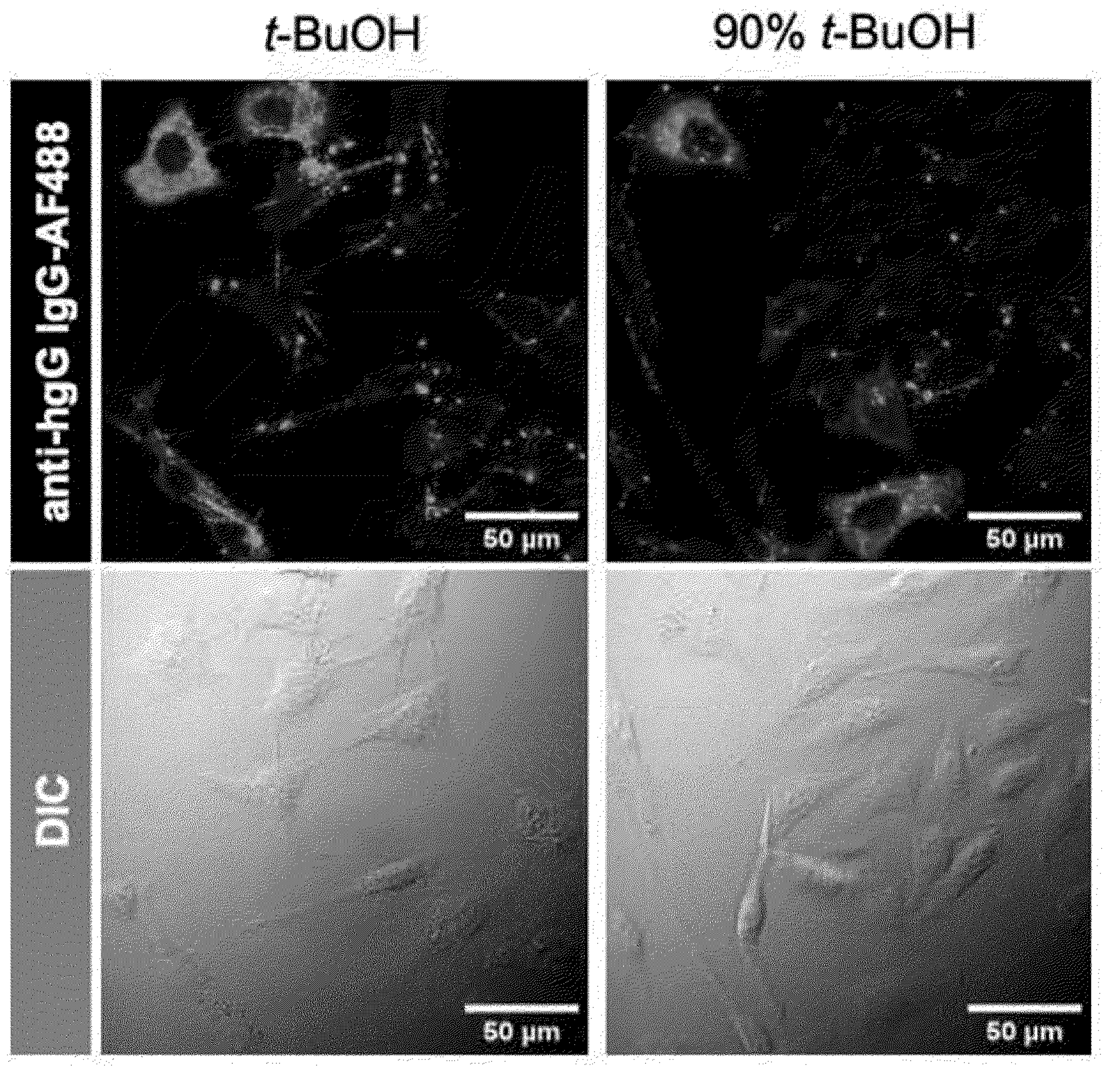

[FIG. 10a]

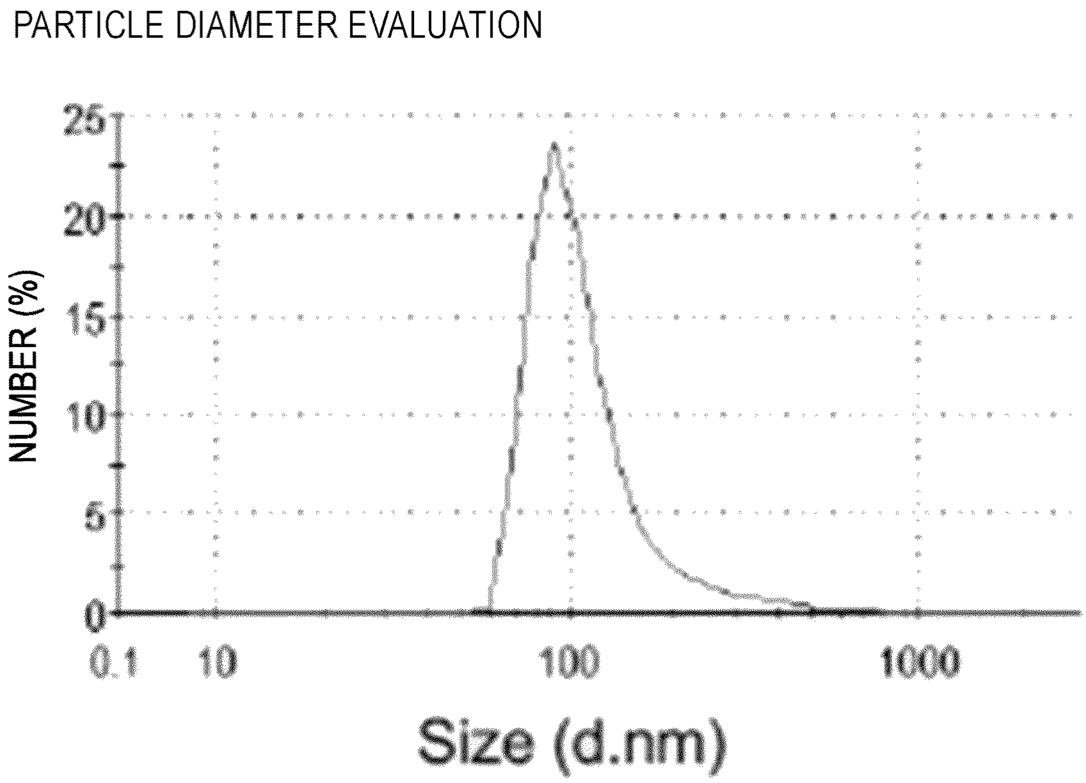
PARTICLE DIAMETER EVALUATION
NUMBER (%)
25
20
15
10
5
0
0.1
10
100
1000
Size (d.nm)

[FIG. 10b]

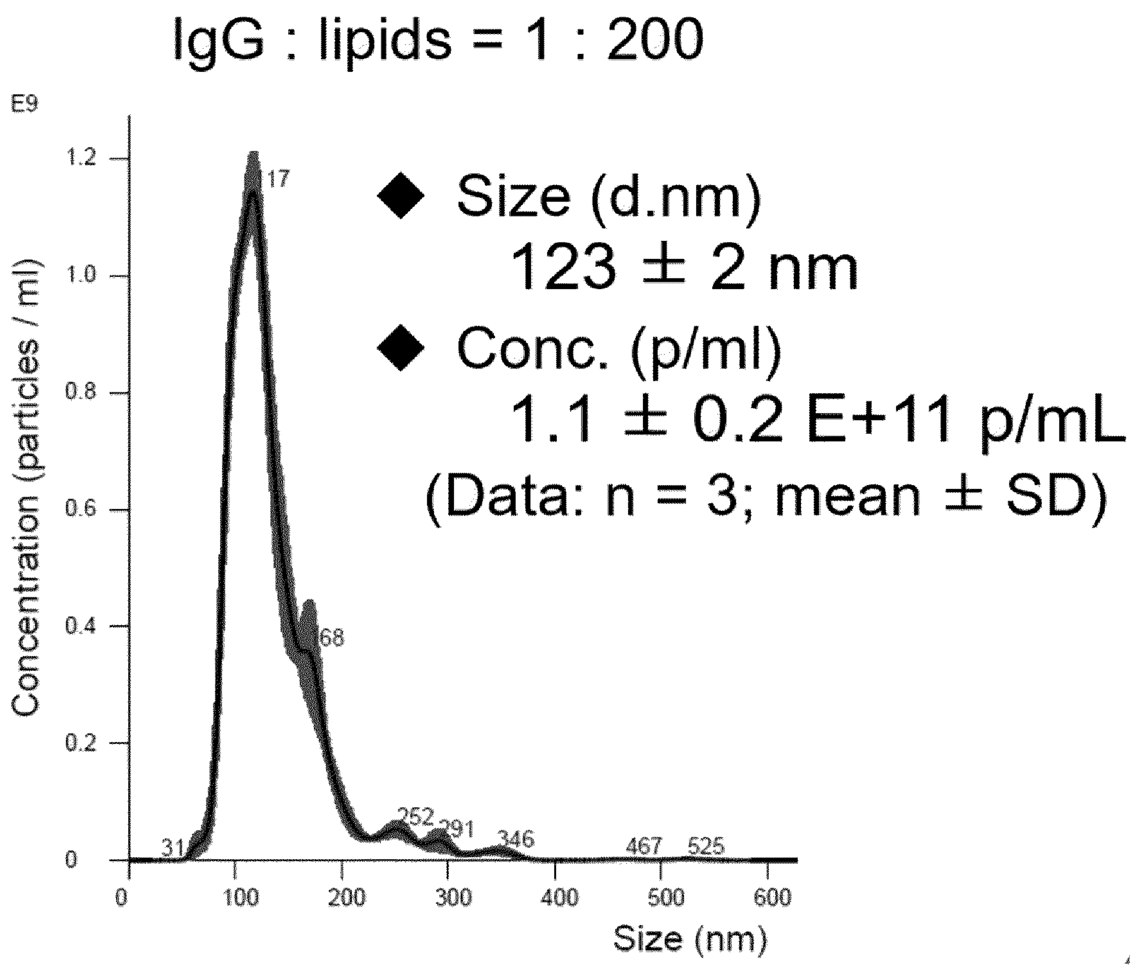
IgG : lipids = 1 : 200
E9
Concentration (particles / ml)
Size (nm)
◆ Size (d.nm)
123 ± 2 nm
◆ Conc. (p/ml)
1.1 ± 0.2 E+11 p/mL
(Data: n = 3; mean ± SD)

[FIG. 11]

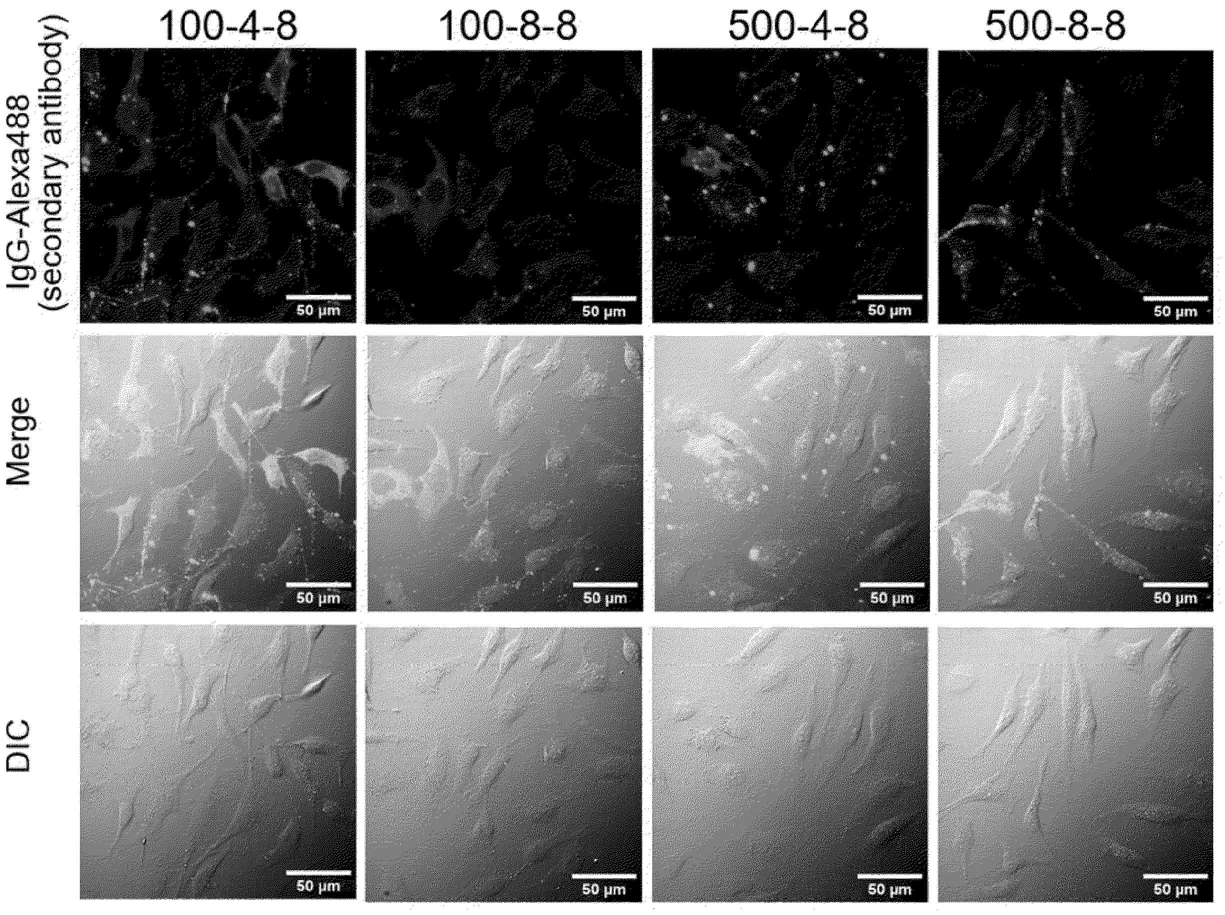
100-4-8
100-8-8
500-4-8
500-8-8
IgG-Alexa488 (secondary antibody)
Merge
DIC
50 µm

[FIG. 12]

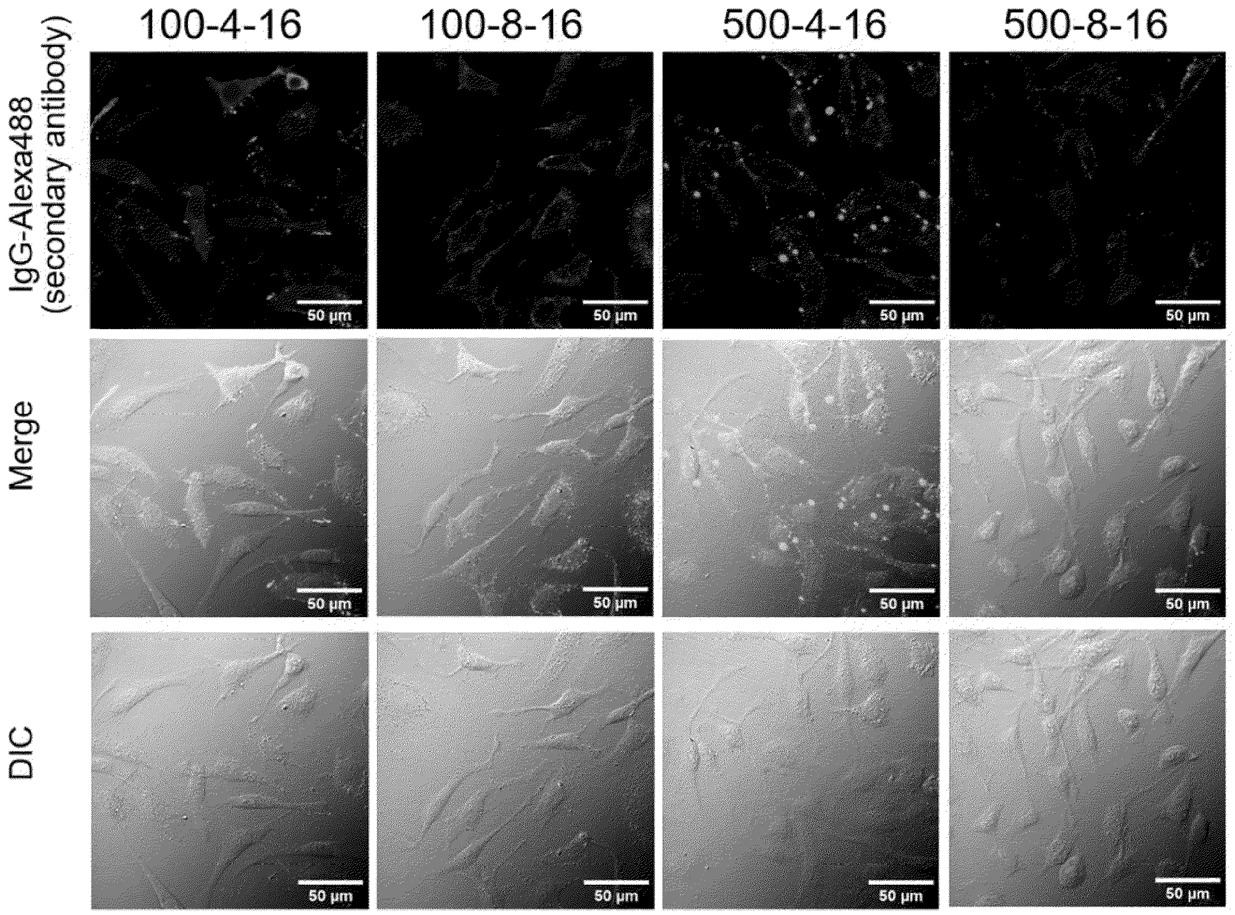
100-4-16
100-8-16
500-4-16
500-8-16
IgG-Alexa488 (secondary antibody)
Merge
DIC
50 μm

[FIG. 13]

| Sample | Z average (d.nm) | PdI |
|---|---|---|
| 100 – 4 – 8 | 317 ± 4.6 | 0.420 ± 0.005 |
| 100 – 8 – 8 | 196 ± 1.1 | 0.211 ± 0.006 |
| 500 – 4 – 8 | 160 ± 2.4 | 0.339 ± 0.02 |
| 500 – 8 – 8 | 166 ± 3.3 | 0.315 ± 0.05 |
| 100 – 4 – 16 | 383 ± 3.1 | 0.441 ± 0.01 |
| 100 – 8 – 16 | 259 ± 5.6 | 0.242 ± 0.01 |
| 500 – 4 – 16 | 130 ± 2.8 | 0.240 ± 0.005 |
| 500 – 8 – 16 | 144 ± 0.91 | 0.240 ± 0.01 |

[FIG. 14]

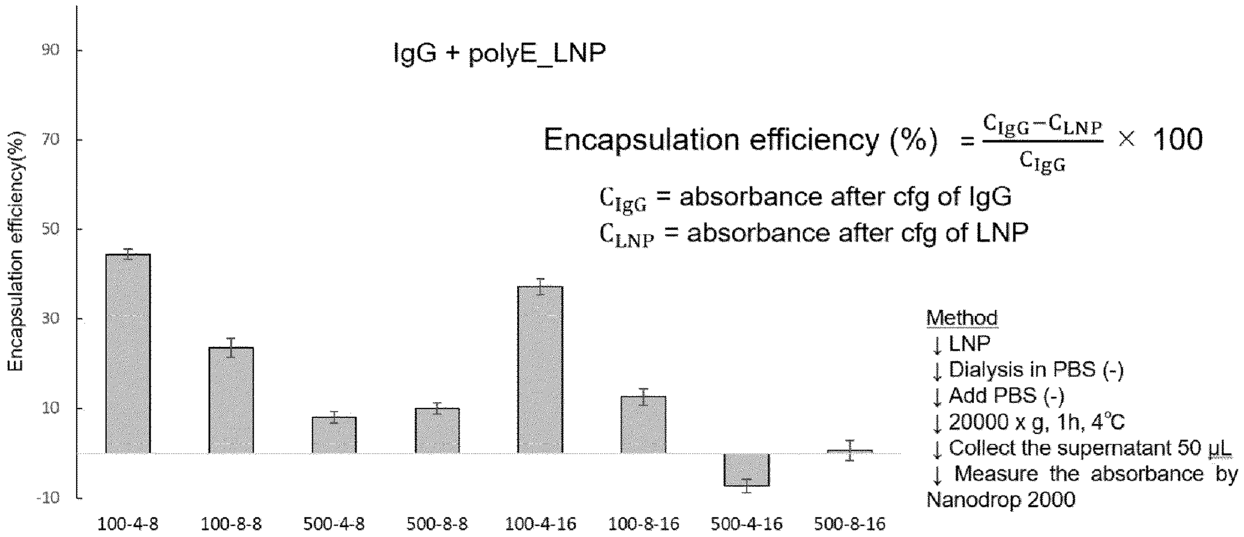


| Sample | Encapsulation efficiency (%) | Sample | Encapsulation efficiency (%) |
|---|---|---|---|
| 100-4-8 | 44.5 ± 1.2 | 100-4-16 | 37.2 ± 1.7 |
| 100-8-8 | 23.6 ± 2.2 | 100-8-16 | 12.6 ± 1.9 |
| 500-4-8 | 8.2 ± 1.3 | 500-4-16 | -7.2 ± 1.6 |
| 500-8-8 | 10.1 ± 1.2 | 500-8-16 | 0.78 ± 2.4 |

[FIG. 15]

| 3-15k polyE | | |
|---|---|---|
| polyE:IgG | Z average (d.nm) | PdI |
| 1:10 | 278 ± 4.0 | 0.310 ± 0.020 |
| 1:5 | 294 ± 0.85 | 0.354 ± 0.018 |
| 2:5 | 269 ± 4.0 | 0.343 ± 0.010 |
| 4:5 | 591 ± 31 | 0.444 ± 0.032 |

| 39k | | |
|---|---|---|
| polyE:IgG | Z average (d.nm) | PdI |
| 1:10 | 311 ± 6.0 | 0.296 ± 0.040 |
| 1:5 | 314 ± 7.5 | 0.294 ± 0.032 |
| 2:5 | 330 ± 25 | 0.299 ± 0.014 |
| 4:5 | 343 ± 8.0 | 0.03 ± 0.002 |

| 2.6k | | |
|---|---|---|
| polyE:IgG | Z average (d.nm) | PdI |
| 1:10 | 313 ± 7.5 | 0.353 ± 0.005 |
| 1:5 | 311 ± 7.6 | 0.371 ± 0.031 |
| 2:5 | 296 ± 7.1 | 0.311 ± 0.059 |
| 4:5 | 349 ± 6.7 | 0.338 ± 0.039 |

[FIG. 16]

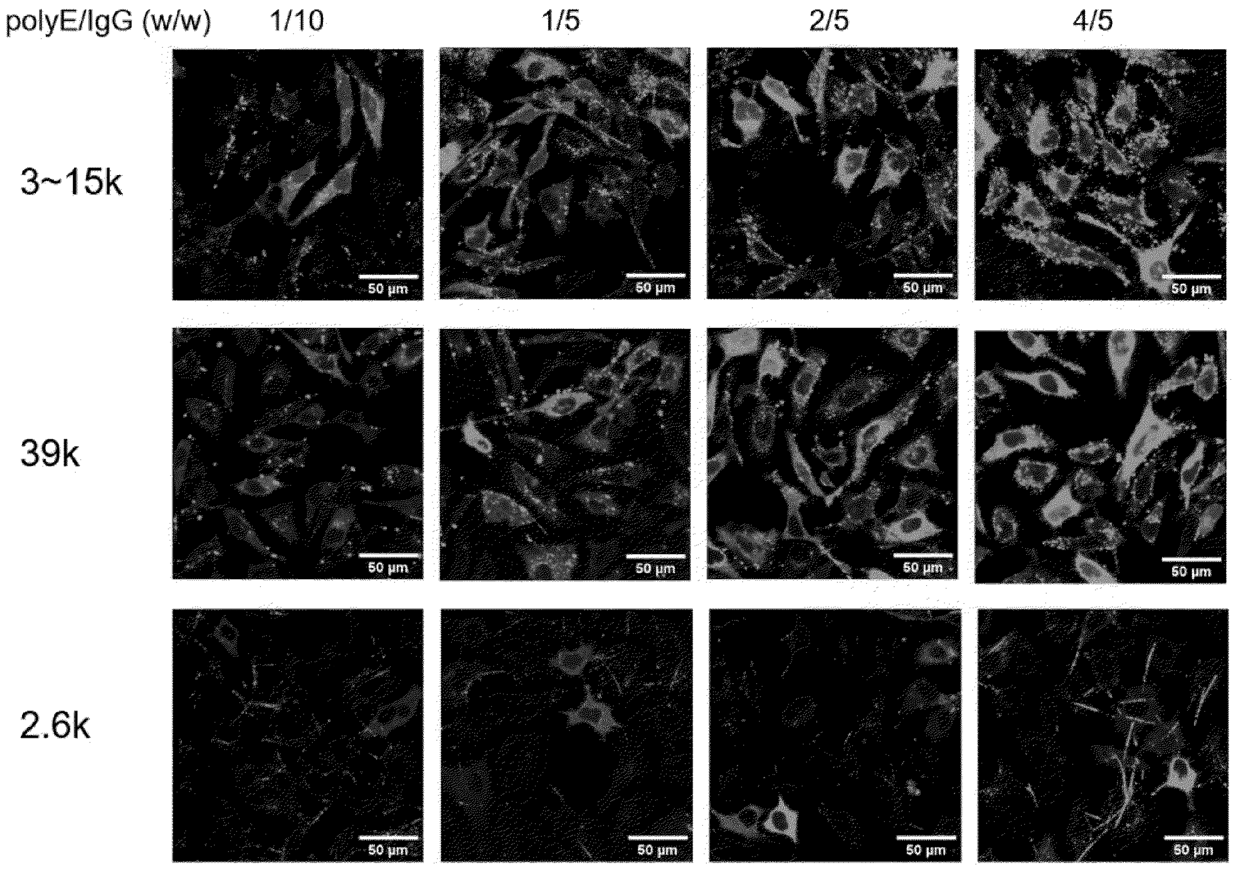

[FIG. 17]

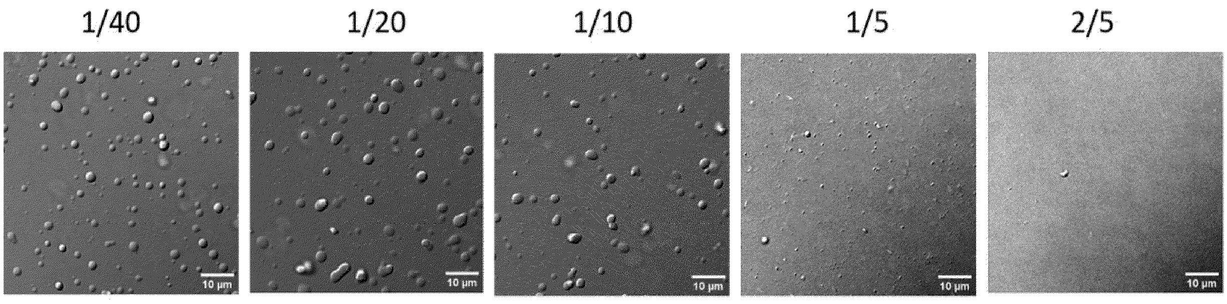


*VALUES ON EACH DRAWING INDICATE HYALURONIC ACID/IgG (w/w)

[FIG. 18]

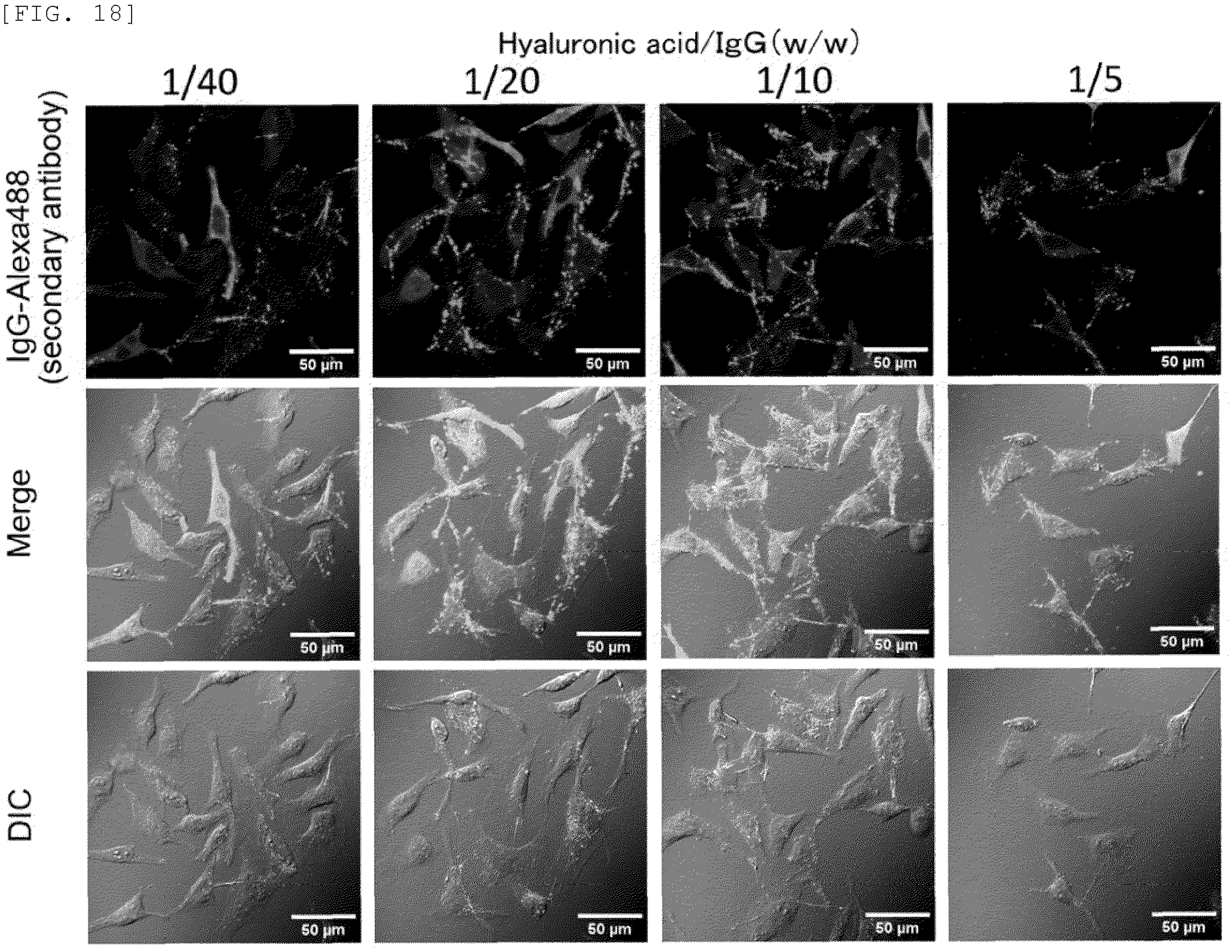
Hyaluronic acid/IgG(w/w)
1/40
1/20
1/10
1/5
IgG-Alexa488 (secondary antibody)
Merge
DIC
50 μm

**INTERNATIONAL SEARCH REPORT**

International application No.
**PCT/JP2024/008154**

**A. CLASSIFICATION OF SUBJECT MATTER**

***A61K 39/395***(2006.01)i; ***A61K 9/51***(2006.01)i; ***A61K 9/127***(2006.01)i; ***A61K 47/14***(2017.01)i; ***A61K 47/24***(2006.01)i; ***A61K 47/28***(2006.01)i; ***A61K 47/36***(2006.01)i; ***A61K 47/42***(2017.01)i; ***A61P 35/00***(2006.01)i; ***A61P 43/00***(2006.01)i
FI: A61K39/395 M; A61K9/127; A61K9/51; A61K47/14; A61K47/24; A61K47/28; A61K47/36; A61K47/42; A61P35/00; A61P43/00 105

According to International Patent Classification (IPC) or to both national classification and IPC

**B. FIELDS SEARCHED**

Minimum documentation searched (classification system followed by classification symbols)

A61K39/395; A61K9/127; A61K9/51; A61K47/14; A61K47/24; A61K47/28; A61K47/36; A61K47/42; A61P35/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | HIRAI, Yusuke et al. Cytosolic protein delivery using pH-responsive, charge-reversible lipid nanoparticles. Scientific Reports. 2021, Vol. 11:19896, pp. 1-13<br>abstract, fig. 1, p. 10, column "Preparation of lipid nanoparticle (LNP)-encapsulated NLS-(- 30)GFP.", p. 2, column "Formulation of charge-reversible lipid-based nanoparticles encapsulating a negatively charged green fluorescent protein.", 1st to 2nd paragraphs | 1-4, 6-11 |
| Y | 平井勇祐ほか, 脂質パッケージングによる負電荷タンパク質の細胞内送達, 日本薬学会　第１４１年会　要旨集（WEBONLY）, 2021, column pm03S, (HIRAI, Yusuke et al. Intracellular delivery of negatively charged protein using lipid-based carrier.), non-official translation (Abstracts of the 141st Annual Meeting of the Pharmaceutical Society of Japan)<br>1st to 2nd paragraphs | 1-4, 6-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

## INTERNATIONAL SEARCH REPORT

International application No.
**PCT/JP2024/008154**

### C. DOCUMENTS CONSIDERED TO BE RELEVANT

| Category* | Citation of document, with indication, where appropriate, of the relevant passages | Relevant to claim No. |
|---|---|---|
| Y | TSUMURA, Keisuke et al. Lowering the viscosity of a high-concentration antibody solution by proteinepolyelectrolyte complex. Journal of Bioscience and Bioengineering. 08 October 2021, vol. 133(1), pp. 17-24<br>fig. 7, abstract | 1-4, 6-11 |
| Y | LU, Tiemei et al. Endocytosis of Coacervates into Liposomes. J. Am. Chem. Soc. 2022, vol. 144, pp. 13451-13455<br>abstract, p. 13451, left column, line 1-right column, line 24, fig. 1 | 1-4, 6-11 |
| Y | ZHANG, Yanwen et al. Giant Coacervate Vesicles As an Integrated Approach to Cytomimetic Modeling. J. Am. Chem. Soc. 2021, vol. 143, pp. 2866-2874<br>abstract, fig. 1 | 1-4, 6-11 |
| X | JP 2010-536786 A (AMGEN INC.) 02 December 2010 (2010-12-02)<br>claims, examples, paragraph [0105] | 1-5, 7-11 |
| A | JP 2021-519748 A (THE JOHNS HOPKINS UNIVERSITY) 12 August 2021 (2021-08-12)<br>entire text, all drawings | 1-11 |
| P, X | HIRAI, Yusuke et al. Liquid Droplet-Mediated Formulation of Lipid Nanoparticles Encapsulating Immunoglobulin G for Cytosolic Delivery. Mol. Pharmaceutics. 30 January 2024, vol. 21, pp. 1653-1661<br>whole document | 1-4, 6-11 |

Form PCT/ISA/210 (second sheet) (July 2022)

**INTERNATIONAL SEARCH REPORT**
**Information on patent family members**

International application No.
**PCT/JP2024/008154**

| Patent document cited in search report | Publication date (day/month/year) | Patent family member(s) | Publication date (day/month/year) |
|---|---|---|---|
| JP 2010-536786 A | 02 December 2010 | WO 2009/026122 A1<br>claims, examples, paragraph [0122]<br>EP 2190476 A1 | |
| JP 2021-519748 A | 12 August 2021 | US 2021/0030692 A1<br>whole document<br>EP 3746056 A1<br>KR 10-2020-0126976 A<br>CN 111954522 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015064591 A
- WO 2019188867 A
- US 4816567 A
- JP 2022122871 A
- JP 5361738 B


### Non-patent literature cited in the description


- **CHATIN B** ; **MEVEL M** ; **DEVALLIERE J et al.** Liposome-based formulation for intracellular delivery of functional proteins. *Mol Ther Nucleic Acids.*, 2015, vol. 4, e244
- Intracytoplasmic delivery of anionic proteins. *Mol Ther.*, June 2004, vol. 9 (6), 964-9
- **KOHLER** ; **MILSTEIN**. *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- *N. Biotechnol.*, 2011, vol. 28 (5), 253-457
- *Journal of Biomedical Science*, 2020, vol. 27, 1
- *Cell*, 2022, vol. 185 (15), 2789-2805
- *Mol. Pharmaceutics*, 2016, vol. 13, 1915-1926
- *Mol. Pharmaceutics*, 2015, vol. 12 (9), 3272-3281
- *Journal of Chromatography B*, 2017, vol. 1065 (1066), 119-128
- **MASHIRO MIMURA**. *J. Chem. Phys.*, 2019, vol. 150, 064903
- *J. Chem. Phys.*, 2019, vol. 150, 064903